# EUROPEAN PATENT SPECIFICATION

(11) **EP 3 684 396 B1**
(45) Date of publication and mention of the grant of the patent: **02.12.2026**
(21) Application number: 18788847.4
(22) Date of filing: 11.10.2018
(51) Int. Cl.: A61K 38/39, C07K 14/78

(54) **SINGLE ALPHA CHAIN COLLAGENS**
EINKETTIGE ALPHA-KOLLAGENE
COLLAGÈNES À CHAÎNE ALPHA UNIQUE

(30) Priority: 18.10.2017 GB 201717134
(43) Date of publication of application: 29.07.2020
(73) Proprietor: Swansea University, Swansea SA2 8PP (GB)
(72) Inventor: WRIGHT, Chris, Swansea SA2 8PP (GB); WIDDOWSON, Jonathan, Swansea SA2 8PP (GB)
(74) Representative: Symbiosis IP Limited
(86) International application number: PCT/GB2018/052913
(87) International publication number: WO 2019/077312

(56) References cited:
- WO-A1-2017/122216
- G CHANDRAKASAN ET AL: "Preparation of intact monomeric collagen from rat tail tendon and skin and the structure of the nonhelical ends in solution", JOURNAL OF BIOLOGICAL CHEMISTRY, 10 October 1976 (1976-10-10), United States, pages 6062 - 6067, XP055529930, Retrieved from the Internet <URL:http://www.jbc.org/content/251/19/6062.full.pdf> [retrieved on 20181210]
- R FLEISCHMAJER ET AL: "Rotary shadowing of collagen monomers, oligomers, and fibrils during tendon fibrillogenesis.", JOURNAL OF HISTOCHEMISTRY AND CYTOCHEMISTRY, vol. 39, no. 1, 1 January 1991 (1991-01-01), US, pages 51 - 58, XP055530182, ISSN: 0022-1554, DOI: 10.1177/39.1.1983873
- IVAN J. HALL BARRIENTOS ET AL: "Electrospun collagen-based nanofibres: A sustainable material for improved antibiotic utilisation in tissue engineering applications", INTERNATIONAL JOURNAL OF PHARMACEUTICS, vol. 531, no. 1, 1 October 2017 (2017-10-01), NL, pages 67 - 79, XP055532814, ISSN: 0378-5173, DOI: 10.1016/j.ijpharm.2017.08.071

## Description

The invention relates to a method for the production of single, alpha chain collagens and an isolated or purified product comprising single alpha chain collagens.

### Background of Invention

Collagens are the main group of structural proteins to be found in the extracellular matrix (ECM), and are the most abundant proteins found in animals, accounting for 25-35% of the whole body protein. Collagens all share a characteristic triple helix structure of Gly-X-Y repeats (where X can be any amino acid, and Y is often proline or hydroxyproline).

So far, 29 different Types of collagens have been classified (Brodsky B. et al; Kielty C. M. et al; Veit G. et al; and Shoulders M. D. et al) arising due to slight sequence differences in the primary structure. However, all collagens consist of three distinct polypeptide α-chains arranged in a triple helical conformation. The proper folding of each of these chains requires a glycine residue to be present at every third position in the polypeptide chain. The presence of proline or hydroxyproline in the Y position is also thought to contribute to the stability of the helical form. The individual chains are wound around one another to form a right handed superhelix rendering collagen fibres insoluble with high tensile strength.

Type I collagen is a heterotrimer comprising two α1 chains and one α2 chain.

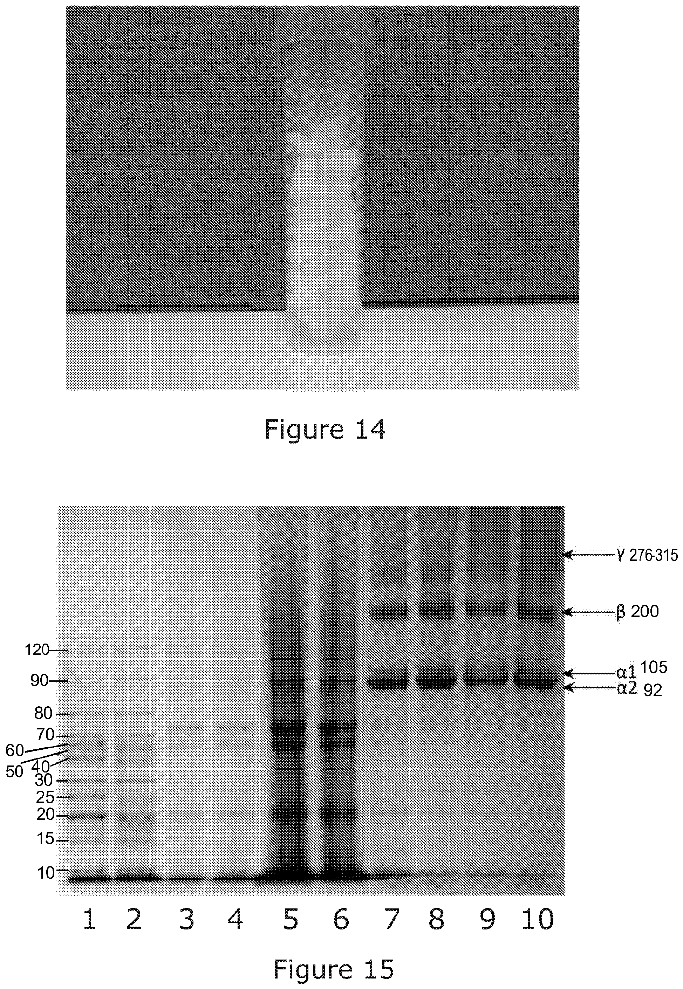

When a tissue-extracted type I collagen sample is denatured and applied to SDS-PAGE, three regions are apparent on the gel: alpha, beta and gamma regions. The alpha region has a molecular weight of approximately 100KDa, the beta region a molecular weight of approximately 200KDa and the gamma region a molecular weight of approximately 300KDA. The alpha region comprises two α1 and one α2 chains, per collagen molecule. The two α1 chains are overlain and produce more intense bands than the one α2 chain. The beta region comprises two combined α1 chains and a combined α1 and α2 chain; these two bands are overlapped on the gel. The gamma region comprises two α1 chains combined with one α2 chain. This is shown in figure 15. Thus, collagen contains a number of α chains that separate on SDS gels in a distinct manner.

Collagen proteins have wide ranging applications, such as the fabrication of scaffolds which are used in applications ranging from 3D cell culture and wound dressings to artificial tissue and organ production.

For collagen to be obtained in a usable form, it is typically extracted from a mammalian source but, more recently, it has been obtained from other sources such as Jellyfish. The standard procedure for obtaining a usable collagen mixture involves a technique of solubilisation in acetic acid (AcOH), which can be accompanied by pepsin treatment, where necessary, to aid in both the speed of extraction and yield of the resulting product. However, the use of pepsin in the process results in collagen which is lacking a significant amount of its telo-peptide region (collagen N- and C-terminal regions, or non-helical ends, important in fibril cross-linking and fibrillogenesis), often termed 'atelo collagen' which can affect its ability to form fibrils thus limiting its usefulness. These extraction processes have been adapted and modified in many ways, though yield has never been shown to rise above 2% from wet weight or 20% dry weight. Further, all extraction methods to date do not provide for the isolation of pure alpha chain collagen extract but rather they provide for alpha chains contaminated with other combined or helical forms of collagen (i.e. as seen as beta and, even more relevant, gamma regions on SDS gel) which can limit downstream utility, for example electrospinning.

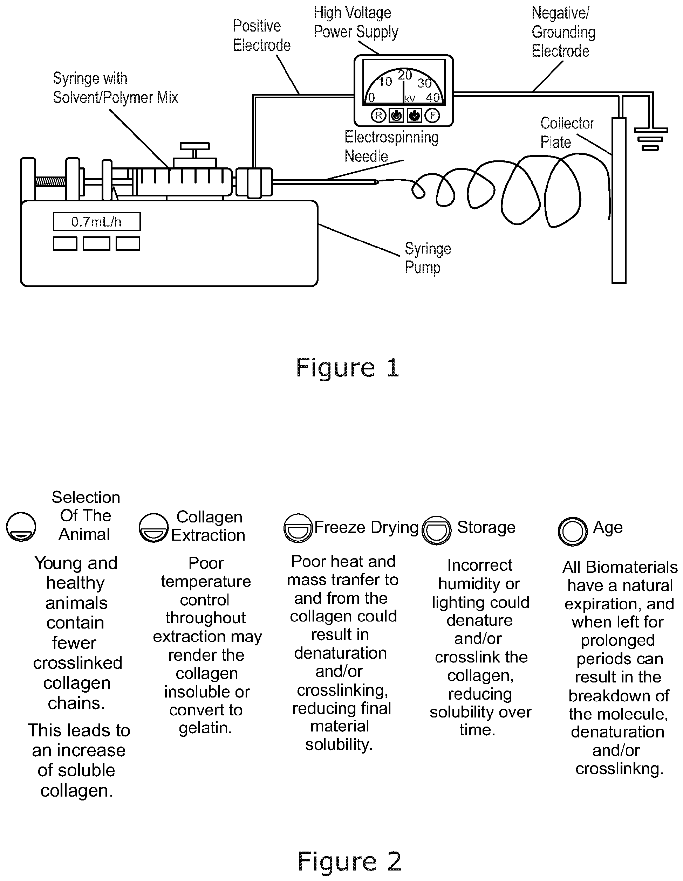

Electrospinning of soluble collagens provides a suitable way of producing 3D scaffolds that closely mimic the high porosity and surface area often seen in the extra cellular matrix (ECM) of tissues. Thus, this fabrication process has been used in the production of scaffold devices for use in applications such as skin grafts and bone repair. The process of electrospinning uses a high voltage power supply to supply a positive charge to an electrospinning needle and a grounding electrode coupled to a collector plate (Figure 1). The electrospinning needle comprises a syringe loaded with polymer and solvent, which is fed at a controlled rate using a syringe pump.

Research into collagen electrospinning has received extra attention in recent years due to the finding that the primary solvents used to electrospin collagen, namely 1,1,1,3,3,3 Hexafluoro-2-Propanol (HFP) and 2,2,2-Trifluoroethanol (TFE) have been shown to denature collagen. As a result many researchers now avoid electrospinning of both natural and synthetic polymer materials because of the reliance on aggressive solvents. There have been attempts to try and electrospin collagen, and other biopolymers, using benign solvent mixtures, however, many of these have been unable to replicate the prior success, in terms of fibre morphology and homogeneity, that collagen electrospun using HFP and TSE produces.

In addition to the concern about the use of solvents, the quality of the extracted collagen also affects how well the solution can be electrospun, for example, there are issues surrounding the electrospinning of gamma collagen, relating to the extraction conditions - these are critical issues that must be addressed. Figure 2 shows how the collagen product can be influenced by a number of extraction conditions.

There is therefore a need for an alternative method of collagen extraction and preparation in order to obtain more stable and pure forms of collagen that are a faithful replication of the natural form and that lend themselves to subsequent manipulation, including, but not limited to, electrospinning.

We therefore herein disclose a novel collagen extraction technique that permits the isolation of pure single alpha chain (α1 & α2) collagens (SACCs) which hitherto has not been possible. These single collagen alpha chain isoforms are shown to be those chains that, importantly, undergo entanglement to form fibres during electrospinning, while the β and γ chains have a rigid structure which cannot form nanofibers. These SACCs have stable non-helical ends (or linear ends), often lost during the extraction techniques of the prior art, that also improve downstream utility. Moreover, the extracted collagen has also been found to be extremely soluble, even in benign solvents such as buffered solution, to an extent not remotely possible with less pure forms of collagen. This work also suggests that the increased solubility of the single alpha chain collagens aids electrospinning and prevents separation of insoluble collagen from solution.

We have shown that isolated SACCs can be electrospun preserving the nature of collagen's fibril forming abilities, while producing nanofiber scaffolds that are suitable for regenerative medicine and tissue engineering applications. We believe that the isolation of SACCs is extremely significant with wide spread application in the improved handling and application of collagen for the fabrication of medical and cosmetic materials.

### Statements of Invention

The present invention, in its various aspects, is as set out in the accompanying claims.

According to a first aspect or embodiment of the invention there is provided a method for the production or purification of single alpha chain collagens (SACCs) comprising:
a) obtaining an extraction of collagen in alkaline solution;
b) filtering the extraction using at least one first filter membrane having a Nominal Molecular Weight Cut-Off (NMWCO) in the range of 1 - 100,000 to produce a first filter retentate;
c) adjusting the pH of the first filter retentate so that it is acidic pH of less than 3 thereby at least partially solubilising the collagen bound by same;
d) filtering the at least partially solubilised first filter retentate until a pH of greater than 6 is achieved using said at least one first filter membrane having a Nominal Molecular Weight Cut-Off (NMWCO) in the range of 1 - 100,000 to produce a second filter retentate;
e) adjusting the pH of the second filter retentate to have a pH of less than 4 thereby solubilising the collagen bound by same;
f) filtering the second filter retentate using at least one second filter membrane wherein the second filter membrane is a micro-porous membrane comprising a pore size between about 0.05 to 2µm and every 0.01µm integer therebetween to produce a first filtrate comprising SACCs; and
g) optionally, filtering the first filtrate using said at least one first filter membrane to produce a second retentate comprising single alpha chain collagen.

Reference herein to retentate refers to the matter retained by the membrane and not allowed to pass through same.

Reference herein to collagen refers to a triple-helical structure based on a (Gly-Xaa-Yaa)n repeating sequence, such as any of the 28 types of collagens that so far have been described, including but not limited to: fibrillary type I collagen comprising two α1(I) chains and one α2(I) chain; fibrillary type II collagen comprising three α1 (II) chains and fibrillary type V collagen comprising three alpha chains (I, II and III).

Reference herein to single alpha chain collagen (SAC) is reference to α1 and α2 chains i.e. single α1 chain or single α2 chain. Further, in the context of collagen type V, this may also further include an α3 chain. As will be appreciated by those skilled in the art, typically α1 and α2 chains are formed during translation on ribosomes along the rough endoplasmic reticulum inside the cell (known as preprocollagen), and form an alpha helix having non-helical ends. However, preprocollagen undergoes cellular processing wherein non-helical ends are cleaved, along with hydroxylation and glycosylation of various amino acids of the alpha chains, leading to formation of what are known as alpha chain propeptides, which then further twist into a triple helix forming procollagen. This process is typically rapid. However, according to the present invention we have isolated these single alpha helix chains from the helix of triple helical collagen, which comprise intact non-helical ends that facilitate re-fibrillation back to triple helix under defined conditions.

As is known to those skilled in the art, NMWCO, or Molecular weight cut-off (MWCO), is a method of characterization used in filtration to describe pore size distribution and retention capabilities of membranes. It is defined as the lowest molecular weight (in Daltons) at which greater than 90% of a solute with a known molecular weight is retained by the membrane. As will be appreciated by those skilled in the art, the exact filter cut off will vary according to the species origin of the collagen being extracted (e.g. mammalian versus cnidaria) and so can be tailored accordingly. More preferably still, the first filter membrane has a NMWCO of 10,000-60,000. Most ideally, the first filter membrane has a NMWCO selected from 10,000 or 50,000.

In the above method, in particular step a), the original collagen containing sample is treated with alkaline or basic solution to allow cellular matter and other non-collagenous material to be destroyed and, in particular, the hydrolysis of fats and proteins which can then be removed through the membrane filtration system. As will be appreciated by those skilled in the art, an alkaline or basic solution is any solution whose pH lies between 7 to 14 and is well known it the art such as, but not limited to, any hydroxide solutions for example ammonium hydroxide, sodium hydroxide, potassium hydroxide, magnesium hydroxide or the like. Preferably, said alkali solution is sodium hydroxide.

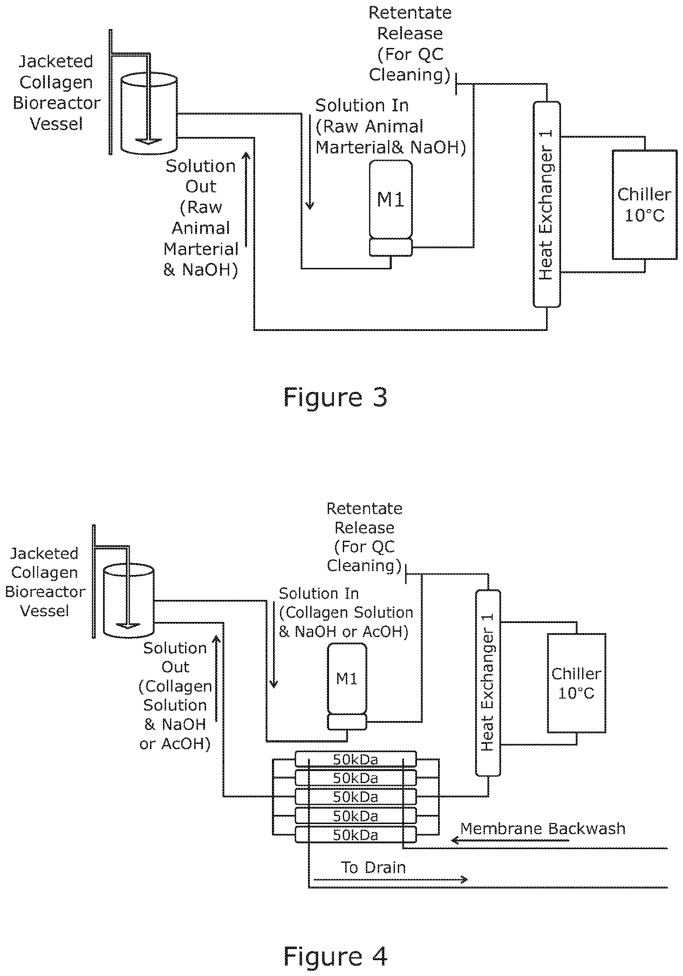

In the above method, in a preferred embodiment, and in particular in step a), said collagen is provided in an alkaline/basic solution to allow cellular matter and other non-collagenous material to be destroyed, preferably at a sample/solution ratio of about 1:1 (w/v) to about 1:100 (w/v) and preferably at a sample/solution ratio of about 1:10 (w/v). Most preferably, the extraction is undertaken by circulation in a closed system, e.g. in a loop such as a circulation loop as illustrated in figure 3 for several days up to and including 3 days.

Preferably, in step b), the extraction is filtered using at least one 50,000 NMWCO hollow fibre membrane. Most preferably the extraction is filtered by circulation in a closed system, e.g. in a loop such as a circulation loop as illustrated in figure 4 to decrease the pH by filtering the basic/alkaline solution. Ideally, the extraction is filtered until a pH <8 is achieved and a first filter retentate is obtained.

As will be appreciated by those skilled in the art, due to the process of filtering there may be a gradual loss of solution volume and as such in a preferred embodiment of the method, in particular in step b) the first filter retentate is supplemented, before during or after filtering, with deionised water to maintain the original volume or the volume required for the execution of the subsequent method steps, so as to achieve optimum desired flow rate, recirculation volumes and the like according to the filter separation system.

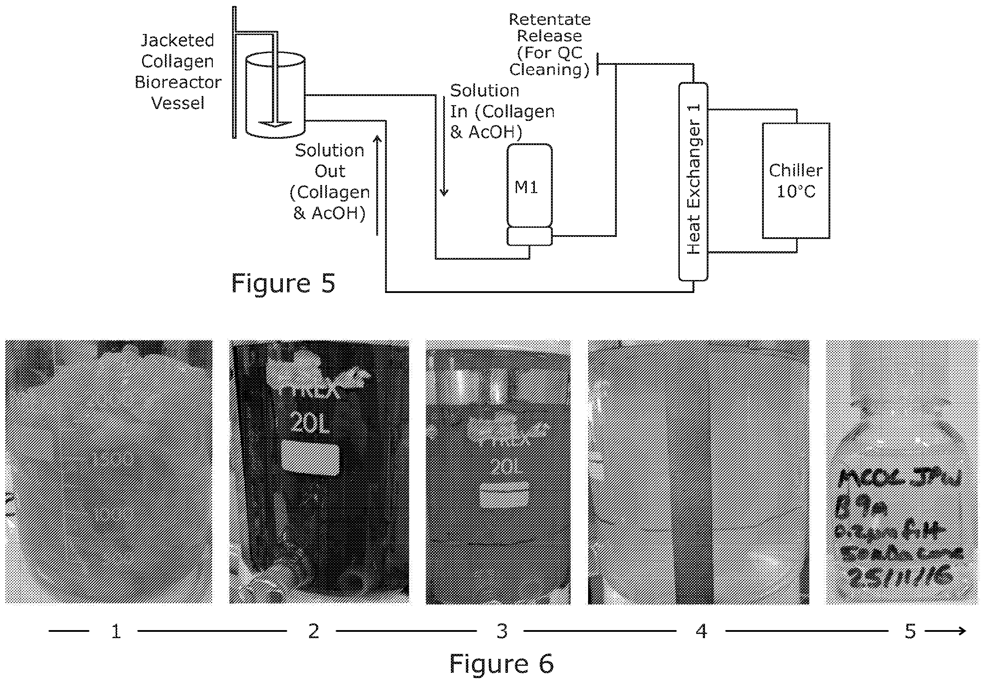

Preferably, in step c), the first filter retentate is adjusted to make a solution having an essentially acidic pH of less than 3. As will be appreciated by those skilled in the art, this can be achieved by supplementing with acidic solutions known in the art such as, but not limited to, acetic acid. Most preferably, the resultant acidified first filter retentate is then circulated in a closed system, e.g. in a loop such as a circulation loop as illustrated in figure 5 for several days at least up to and including 3 days, more preferably at least up to and including 7 days. As will be appreciated by those skilled in the art, this step facilitates the digestion of intramolecular bonds of the collagen fibrils and leads to partial cleavage of the telopeptide regions and thus partially solubilising the collagen.

Preferably, in step d), the first filter retentate is filtered to remove the acid until a pH of greater than 6 is achieved, and this preferably comprises the use of at least one 50,000 NMWCO hollow fibre membrane to obtain a second filter retentate. Ideally, this is undertaken in a circulation loop such as a circulation loop as illustrated in figure 4.

As will be appreciated by those skilled in the art, due to the process of filtration there may be a gradual loss of solute volume and where this occurs, in a preferred embodiment of step d), the second filter retentate is supplemented with deionised water preferably to maintain the original volume or the volume required for the execution of the subsequent method steps, so as to achieve an optimum desired flow rate, recirculation volumes and the like according to the filter separation system.

**In** yet a further preferred method of the invention, steps c) - d) are repeated at least once to improve yield and purity.

**In** a yet a further preferred method of the invention still, steps c) - d) are performed in a closed circulatory system that either runs constantly or runs constantly during the performance of the said specified steps.

In yet a further preferred embodiment of the invention said filtering involves the process of dialysis.

Those skilled in the art will appreciate that steps a-d may be omitted when using an acid soluble source of collagen. Accordingly as taught herein the method simply involves the following steps:
e) adjusting the pH of the second filter retentate so that it is acidic thereby solubilising the collagen bound by same;
f) filtering the second filter retentate using at least one second filter membrane to produce a first filtrate comprising single alpha chain collagen; and
g) optionally, filtering the first filtrate using said at least one first filter membrane to produce a second retentate comprising single alpha chain collagen.

Preferably, in step e), the second filter retentate is adjusted to make a solution having an essentially acidic pH of less than 3. As will be appreciated by those skilled in the art, this can be achieved by supplementing with acidic solutions known in the field such as, but not limited to, acetic acid. Most preferably, the resultant acidified second filter retentate is then circulated in a closed system, e.g. in a loop such as circulation loop as illustrated in figure 3. As will be appreciated by those skilled in the art, this further acidification step leads to further cleavage of the intramolecular bonds of the collagen and full solubilisation of any collagen fibres.

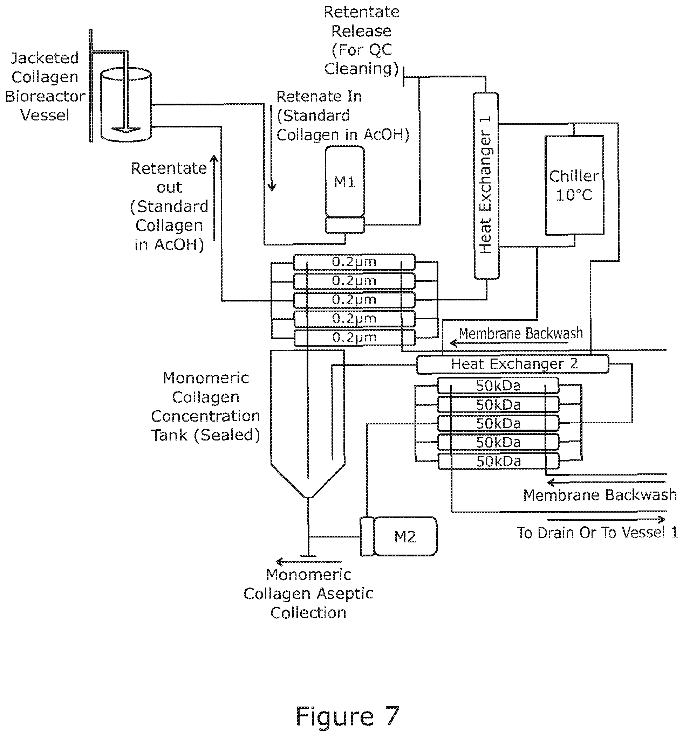

Preferably, in step f), the second filter retentate is filtered through at least one second filter membrane comprising a pore size in the range of 0.05 to 2µm, including every 0.01µm integer therebetween to obtain a purified first filtrate. More preferably, the second filter retentate is filtered until a pH of less than 8 is obtained. Yet more preferably the second filter membrane comprises a pore size of in the range of 0.1 to 1µm, including every 0.01µm integer therebetween, is used. Most ideally, a pore size of about 0.2 to 0.5µm is used. Ideally the solution is circulated in a circulation loop such as a circulation loop as illustrated in figure 7.

As will be appreciated by those skilled in the art, due to the process of filtration there may be a gradual loss of solute volume and where this occurs, in a preferred embodiment of step f), the second filter retentate is supplemented with 0.5M acetic acid (this can be 0.0M - 1M as above) preferably to maintain the original volume or a total volume of at least 20% of reactor capacity to increase filtrate yield.

Preferably, in step g), the first filtrate is retained with said at least one first filter, preferably until a pH of less than 8 is achieved. Most preferably at least one 50,000 NMWCO hollow fibre membrane, to concentrate the solution to greater than 0.1mg/ml but preferably 3-10mg/ml and most preferably 6mg/ml.

In a preferred embodiment of the invention, said collagen is of Mammalian or Cnidarian origin, such as ungulate or bovine / fish or jellyfish origin.

In yet a further preferred embodiment, said collagen is any type of collagen known to those skilled in the art such as, but not limited to, Type I, II, III, IV, V, VI, VII, VIII, IX, X, XI, XII, XIII, XIV, XV, XVI, XVII, XVIII, XIX, XX, XXI, XXII, XXIII, XXIV, XXV, XXVI, XXVII, XXVIII, or XXIX. More preferably, said collagen is a fibril forming collagen selected from the group comprising: Type I, II, III, V, XI, XXIV, XXVI, XXVII or XXVIII. Most preferably, said collagen is selected from the group comprising: Type I, II, III, V, XXIV or XXVI.

According to a further aspect of the invention there is provided an isolated or purified product comprising or consisting of single alpha chain collagens (SAC), typically but not exclusively, obtained using one of the afore methods.

In a preferred embodiment, said product has at least 50% SACCs content per total collagen protein content, more ideally at least 75% SACCs content per total collagen protein content, or yet more ideally at least 85% SACCs content, or most ideally, 85%, 86%, 87%, 88%, 89%, 90%, 91%, 92%, 93%, 94%, 95%, 96%, 97%, 98%, 99% or 100% SACCs content per total collagen protein content. Preferably, said product does not comprise beta and/or gamma collagen i.e. double or triple helical chains, respectively.

In yet a further preferred embodiment, said SACCs are characterised by a molecular weight of about 50-150 kDa. More ideally, said SACCs are characterised by a molecular weight of about 80-120 kDa and most ideally about 90-110 kDa.

In yet a further preferred embodiment still, said single alpha chain (SACC) collagen comprises approximately 1000-3500 amino acids, yet more preferably it comprises between 1000-1500 amino acids. As is known by those skilled in the art, different collagen family members have varying amino acid lengths for both alpha1 and alpha2 chains, for example collagen I has chain lengths of 1464 and 1366 amino acids for alpha1 and alpha2, respectively, whereas collagen XXVI has an alpha1 chain length of 3051 amino acids (table 2). Accordingly, the length of the SACC will be determined by the nature of the starting collagen material as will be appreciated by those skilled in the art.

Yet more preferably still, said SACCs comprise N-terminal and/or C-terminal ends that exist in non-helical, or unwound, form, also known for the selective re-assembly of the single alpha chain structures into a double or triple helix and so provides for the potential for the production of double or triple helices from isolated SACCs, having regard to reaction conditions. This includes, for example, PBS, Media or other buffered salt solution at conditions matching physiological pH and salt content, at temperatures from 4-40°C.

As will be appreciated by those skilled in the art, isolation of said product, or SACCs, is characterised by having little or no triple helix present i.e. the single alpha chain collagens have not undergone association, with other single alpha chain collagens, to form a triple helix but rather exists wholly or substantially in a single alpha chain form. Triple helix formation/presence can be tested by numerous means known in the art such as, but not limited to, FTIR spectroscopy wherein SACCs are identified by a reduction of amide I absorbance at wavelength 1638cm-1 compared to triple helix collagen.

More ideally still, the product of the inventive method is suspended in a solution suitable for the purpose of electrospinning. SACCs dissolve in benign solvents (acetic acid) as well as physiological buffers such as PBS, media etc. Indeed, it has been found that SACCs are extremely soluble in benign solvents such that concentrations of up to 98% w/v SACCs have been achieved. In contrast, conventional acid solubilised collagen is, as the name would suggest, poorly soluble in benign solutions and even then, at best, only soluble at concentrations typically (up to) around 10 % w/v, wherein above that amount precipitation/suspension readily occurs. Further, the poor solubility of acid soluble collagen (ASC) also means that electrospinning at higher concentrations of ASC is not possible. Without wishing to be bound by theory, it is thought that the increased solubility of SACCs is owing to the fact that the isolated SACCs lack helical forms of collagen (e.g. beta and or gamma) and also possesses increased free hydrogen bonds. Accordingly, there is provided a benign buffer in which SACCs are dissolved. Advantageously, this solubility is important for applications in tissue engineering / medical devices etc. as the currently used HFP denatures the protein and causes immunological damage to tissues and patients.

Therefore, in yet a further preferred embodiment, said product comprises SACCs that have not been denatured and are dissolved in a benign solution such as but not limited to, physiological buffers, weak solvents, or water. In contrast, collagens isolated by other means, and not of SACCs form, can only be dissolved in such benign solutions following harsh denaturing protocols. In a preferred embodiment, said solution comprises SACCs at a concentration of at least 50% w/v, ideally at least 75% or yet more ideally at least 85% w/v, or most ideally, 85%, 86%, 87%, 88%, 89%, 90%, 91%, 92%, 93%, 94%, 95%, 96%, 97%, 98%, 99% or 100%.

According to a further aspect of the invention there is provided isolated single alpha chain collagens (SACCs) in aqueous solution wherein said SACCs is or has:
a) a single chain polypeptide existing as an alpha helix and having a molecular weight of 80-120kDa;
b) a repeating sub structure of Glycine-X-Y (where X can be any amino acid, and Y is proline or hydroxyproline);
c) non-helical N-terminal and/or C-terminal ends; and further wherein
d) said aqueous solution i) does not include beta or gamma collagen, ii) contains an amount of SACCs per total collagen protein content of at least 75% and iii) comprises SACCs at a concentration of at least 85% w /v.

In a preferred embodiment of the invention, said collagen is of Mammalian or Cnidarian origin, such as ungulate or bovine / fish or jellyfish origin.

In yet a further preferred embodiment, said collagen is any type of collagen known to those skilled in the art such as, but not limited to, Type I, II, III, IV, V, VI, VII, VIII, IX, X, XI, XII, XIII, XIV, XV, XVI, XVII, XVIII, XIX, XX, XXI, XXII, XXIII, XXIV, XXV, XXVI, XXVII, XXVIII, or XXIX. More preferably, said collagen is a fibril forming collagen selected from the group comprising: Type I, II, III, V, XI, XXIV, XXVI, XXVII or XXVIII. Most preferably, said collagen is selected from the group comprising: Type I, II, III, V, XXIV or XXVI.

In yet a further preferred embodiment still, said single alpha chain (SACC) collagen comprises approximately 1000-3500 amino acids, yet more preferably comprises between 1000-1500 amino acids.

In a preferred embodiment, said isolated SACCs have at least 75% SACC per total collagen content,, or yet more ideally at least 85% SACCs per total collagen content, or most ideally, 85%, 86%, 87%, 88%, 89%, 90%, 91%, 92%, 93%, 94%, 95%, 96%, 97%, 98%, 99% or 100% SACCs per total collagen content.

More ideally, said SACCs are characterised by a molecular weight of about 80-120 kDa and most ideally about 90-110 kDa.

As disclosed herein said SACCs comprise N-terminal and/or C-terminal ends that exist in non-helical, or unwound, form, also known for selective re-assembly of the single alpha chain structures into a double or triple helix and so provides for the potential to produce double or triple helices from isolated SACCs, having regard to reaction conditions. This includes, for example, PBS, Media or other buffered salt solution at conditions matching physiological pH and salt content, at temperatures from 4-40°C.

Using our methods the isolation of SACCs occurs with little or no triple helix formation. Therefore, according to a further preferred embodiment said SACCs are characterised by little or no or reduced gamma collagen or triple helix formation. The presence of triple helices or triple helix formation can be tested by numerous means as known in the art such as, but not limited to, FTIR spectroscopy wherein single alpha chain collagen is identified by reduction of amide I absorbance at wavelength 1638cm-1 compared to triple helix collagen.

In a preferred embodiment, said solution comprises SACCs at a concentration of at least 85% w/v, or most ideally, 85%, 86%, 87%, 88%, 89%, 90%, 91%, 92%, 93%, 94%, 95%, 96%, 97%, 98%, 99% or 100%.

According to a further aspect of the invention there is provided the use of said isolated SACCs for electrospinning.

Accordingly, there is also provided a suspension of said isolated SACCs wherein said suspension comprises a solution suitable for electrospinning. Said isolated SACCs electrospin in benign solvents (acetic acid) as well as physiological buffers such as PBS, media etc. This is an important and advantageous feature for tissue engineering applications and medical devices as the HFP used currently in electrospinning denatures the protein and causes immunological damage to tissues and patients. As discussed, conventional extracts of collagen are poorly soluble in such benign aqueous solutions and thus cannot be electrospun in same and, moreover, the presence of gamma collagen in such extracts and the lack of non-helical ends limits their utility.

Therefore, according to a further aspect of the invention, there is provided an electrospinning solution comprising the isolated SACCs as herein described.

Throughout the description and claims of this specification, the words "comprise" and "contain" and variations of the words, for example "comprising" and "comprises", mean "including but not limited to" and do not exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

No admission is made that any reference constitutes prior art. Further, no admission is made that any of the prior art constitutes part of the common general knowledge in the art.

Preferred features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

The invention will now be described, by way of example only, with reference to the following figures and tables**.** The invention is defined by the scope of the claims.
**Figure-1.** A basic electrospinning set up using a syringe pump and high voltage power supply connected with a grounded collector plate;
**Figure-2.** Chart of extraction stages which contain critical points necessary to avoid solubility issues with collagen extracts;
**Figure-3.** Loop 1 in collagen extraction, material is circulated to control temperature at 10°C or lower;
**Figure-4.** Loop 2 in collagen extraction; material is circulated to control temperature and is retained by 1 - 100,000 NMWCO (10kDa representative) hollow fibre membranes to remove NaOH and non-collagenous material from system;
**Figure-5.** Loop 3 collagen circulation through heat exchanger to maintain temperature at or below 10°C while acid solubilisation occurs;
**Figure-6.** Progress of extraction - 1: Raw Starting Material. 2: NaOH Treatment. 3: Dialysis & AcOH Treatment. 4: Second Dialysis and AcOH Treatment. 5: 0.2µm filtered, 50kDa concentrated SACCs;
**Figure-7.** Cycle of collagen treatment through crossflow filtration system to produce SACCs solutions;
**Figure-8.** Collagen powder obtained through traditional extraction on freeze dried jellyfish material;
**Figure-9.** Collagen sponge obtained through traditional extraction on hydrated jellyfish material;
**Figure-10.** (Left to Right) a) Spray Dried Collagen. b) Freeze Dried Collagen Sponge (12mg/mL). c) Freeze Dried Collagen Sponge (6mg/mL). d) Freeze Dried Collagen Sponge (3mg/mL);
**Figure-11.** Membrane Extracted Jellyfish Collagen (12mg/ml);
**Figure-12.** Bovine Collagen Extract (15mg/mL);
**Figure-13.** SACCs (3mg/ml);
**Figure-14.** Lyophilised SACCs Bovine Collagen;
**Figure-15.** SDS PAGE of collagen extracted from *Rhizostomas pulmo* showing collagen bands present in solution. Lanes 1&2: Benchmark High Molecular Weight Protein Ladder. Lanes 3-6: Non-Collagenous Proteins Removed from Solution During Extraction. Lanes 7-10: Collagen Solution Containing α1, α2, β & γ Collagen Chains;
**Figure-16.** a) SDS PAGE of SACCs extracted from *Rhizostomas pulmo* showing collagen bands present in solution. Lanes 1&2: Benchmark High Molecular Weight Protein Ladder. Lanes 3-4: Collagen Solution Containing α1, α2 Collagen Chains with high molecular weight β & γ chains removed. b) SDS PAGE of electrospun SACCs extracted from *Rhizostomas pulmo* redissolved in 0.5M AcOH post electrospinning showing collagen bands present in solution. Lanes 1&2: Benchmark High Molecular Weight Protein Ladder. Lanes 3-4: Collagen Solution Containing α1, α2 Collagen Chains without high molecular weight β & γ chains;
**Figure-17.** Jellyfish SACCs solution being electrospun with constant droplet replenishment and a single taylor cone producing a jet of collagen solution which lands on the grounding target as collagen nanofibers;
**Figure-18.** A) SEM Micrograph of needle electrospun jellyfish collagen fibres using a Hitachi S4800 FEG-SEM at an acceleration voltage of 10kV, emission current of 9µA and magnification of 1500X. Solution composition was 25% collagen *(w*/*v)* in a 90% AcOH solution (v/v). Fibre Diameter is shown to be 646nm ±121nm. Scale Bar = 10µm. B) Frequency of Range of SACCs Nanofibres from Jellyfish Sources from 0.3µm to 1.1µm. with a fibre diameter of 646nm ±121nm;
**Figure-19.** Electrospun Mat of SACCs consisting of pure α1 & α2 collagen chains;
**Figure-20.** Bovine SAC solution being electrospun with constant droplet replenishment and a single taylor cone producing a jet of collagen solution which lands on the grounding target as collagen nanofibers;
**Figure-21.** A) SEM Micrograph of needle electrospun bovine collagen fibres at an acceleration voltage of 10kV, emission current of 9µA and magnification of 1500X. Solution composition was 35% collagen *(w*/*v)* in a 90% AcOH solution (v/v). Fibre Diameter is shown to be 84nm ±27nm. Scale Bar = 1000nm. B) Frequency of Range of SACCs Nanofibres from Bovine Sources; as above range is from 40nm to 270nm with a fibre diameter of 84nm ±27nm;
**Figure-22.** A) SEM Micrograph of needle-less electrospun bovine collagen fibres at an acceleration voltage of 2kV and magnification of 1800X. Solution composition was 25% collagen *(w*/*v)* in a 90% AcOH solution (v/v). Fibre Diameter is shown to be 1128nm ±406nm. Scale Bar = 30µm. B) Frequency of Range of SACCs Nanofibres from Jellyfish Sources Produced with Needle-less Electrospinning Techniques, range is from 0.2µm to 2.4µm with an average fibre diameter of 1128nm ±406nm;
**Figure-23.** A) SACCs electrospun from PBS solution with an acceleration voltage of 5kV and magnification of 10,000X. Solution composition was 20% collagen *(w*/*v)* in a 1X PBS solution. Fibre diameter is shown to be 105nm ± 28nm. Scale Bar = 5µm. B) Frequency of range of SACCs nanofibres electrospun from a solution of 20% collagen in 1X PBS;
**Figure-24.** SEM micrograph of jellyfish SACCs fibres electrospun and subsequently crosslinked using EDC;
**Figure-25.** Comparison of SACCs prior and post electrospinning; Electrospun samples show a reduction in the amide I peak representing a further reduction in any triple helical collagen content;
**Figure-26.** Chart comparing SACCs and membrane extracted collagens to examine any differences in structure. All data was baseline corrected and normalised using Perkin Elmer Spectrum software at 1558cm⁻¹. The SACCs displays a decrease in the amide 1 band region relative to the standard extraction sample, suggesting a reduction in triple helix presence at 1638cm⁻¹; This correlates well with the SDS PAGE results, where the triple helical γ-chain collagens have been removed in SACCs samples;
**Figure-27.** FTIR Spectra comparing collagen extracted from jellyfish with rat tail collagen type I. Black spectra represents jellyfish collagen, red spectra represents rat tail collagen. Homology is 60.4%. This demonstrates that the membrane extracted jellyfish is not affected by the extraction process in comparison to classically extracted collagens such as rat tail;
**Figure-28.** FTIR Spectra comparing membrane bovine collagen extract with alpha chain bovine collagen. Black spectra represents alpha chain bovine collagen, blue spectra represents bovine collagen extracted using the membrane collagen. Homology is 21.8% with a large reduction in the amide I band; this amide I reduction demonstrates the large reduction in triple helical collagen present in the sample. As the standard bovine collagen was insoluble when purchased and underwent minimal processing for solubilisation, there remains a large fraction of β & γ chain collagens which is seen in figure 15. This clearly demonstrates a distinct separation from standard collagen extracts as the SACCs has a distinct FTIR absorbance pattern, sharing a homology of only 21.8% with the solution from which it is derived/ separated. Thus, the FTIR spectra acts as a 'fingerprint' to the protein which is unique. [For 2 samples to be considered identical (such as in the production of paracetamol) a homology value >95% is required, making this a very selective process.]
**Figure-29.** Triple helix peptide of Gly-Pro-Hyp (Glycine-Proline-HydroxyProline) repeats arranged with a hybrid Lui Storey and Conjugate Descent optimisation (LS-CD) using Abalone software to build amino acid structure;
**Figure-30.** Hydrogen bonding analysis between collagen chains is shown in green. The bonding sites face inward on the chains, preventing access to external bonding from water. These sites are exposed in single chain SACCs which lead to an increased solubility, allowing for dissolution in PBS and Hydrophillic solutions in single chain form which cannot be achieved with standard collagen while avoiding the negative attributes of gelatin extracts. Bonding site analysis produced using Zeus software;
**Figure-31.** Single chain peptide of Gly-Pro-Hyp repeats arranged with a hybrid Lui Storey and Conjugate Descent optimisation (LS-CD) using Abalone software to build amino acid structure;
**Figure-32.** Molecular analysis of [A] Acid Soluble Collagen (ASC) and [B] Single alpha chain collagen (SACC). ASC clearly comprises a triple helical structure, whereas SACC is composed of a single helical alpha chain. Moreover, unlike ASC wherein intramolecular bonding within in the triple helical structure leads to less free unoccupied hydrogen bonds, SACC has a much greater free hydrogen bond content per chain leading to improved solubility in benign solvents up to 99% by weight;
**Figure-33.** SDS page of extracted collagen comparing the composition of the acid soluble collagen (ASC) and samples of single alpha chain collagen (SACC). (A). SACC comprises single alpha 1 and alpha 2 chains but is free from contamination with beta and gamma chains. (B) ASC comprises a mixture of alpha 1 and alpha 2 chains, but also high molecular weight beta and gamma chains. (C) ASC when comprising 60% alpha chain composition still has substantial beta and gamma chain contamination that limits downstream utility;
**Figure-34.** SDS PAGE displaying single α-chain collagen (SACC) post-electrospinning with the absence of high MW β & γ chains. In contrast, Acid soluble collagen can only be electrospun harsh solvents such as hexafluoropropanol (HFP) and as a consequence display very little remaining banding due to the chain breakdown caused by HFP; and
**Figure-35.** SEM micrograph of electrospun collagen comparing acid soluble collagen (ASC) and single alpha chain collagen (SACC) in different solvents: [A] 10% ASC in 100% hexafluoropropanol (HFP) following 48h solubilisation; [B] 10% ASC in 90% acetic acid; [C] 10 ASC in 45% Acetic Acid; [D] 25% jellyfish SACC in 90% Acetic Acid; [E] 20% bovine SACC in 1x Phosphate buffered saline. Acid Soluble Collagen (ASC) does not electrospin without the use of HFP or other fluorinated solvent (i.e. TFE) [A-C], and non-denatured (Gamma containing) ASC electrospun with Acetic Acid (AcOH) produces micro/nano spheres (B-C). In contrast, Single alpha chain collagen (SACC) electrospins without the use of HFP or other fluorinated solvents but instead can be achieved using benign solvents such as acetic acid and physiological buffered (PBS) solutions.

**Table 1.** Peak Picking of FTIR absorption values for analysis of corresponding vibrations in secondary structure;
**Table 2.** Typical amino acid chain lengths of alpha1 and/or alpha2 chains of a selection of different collagen molecules

### METHODS & MATERIALS

### Collagen extraction method

### Traditional Acid Solubilisation - Freeze Dried Jellyfish Material

The freeze-dried Jellyfish was thawed at 4°C overnight. This was then cut into pieces of 0.5cm² and washed with distilled water to remove large impurities and rehydrate the specimen. The samples were then treated with 0.1M NaOH (1M Stock, Fisher, UK) at a sample/solution ratio of 1:10 (w/v). This was then stirred using a magnetic stirrer for 2 days at 4°C. The solution was then filtered through cheesecloth, followed by glass wool to remove any insoluble material. The solution was then poured into dialysis tubing and dialyzed against 5L of Dialysis Buffer at 4°C which was changed daily for 3 days. The collagen then began precipitating out of solution and appeared as a white solid. The collagen containing solution was then centrifuged at 10,000rpm / 16,000g for 10 minutes at 4°C. This was then suspended in 500ml 0.15M acetic acid for 48h at 4°C to purify the collagen. 25g of solid NaCl was then added to give a concentration of 5% and overnight incubation at 4°C dissolved the salt, causing the displacement and precipitation of the collagen. The centrifugation through to salting out was repeated twice to further purify collagen, followed by a final centrifugation at 10,000rpm for 10 minutes at 4°C. The final pellet was dissolved in 500mL 4°C 0.15 M Acetic Acid and dialyzed against Dialysis Buffer for 5 consecutive days at 4°C to remove all traces of NaCl, ensuring the Dialysis Buffer was changed daily. The solution was then centrifuged at 10,000rpm / 16,000g for 10 minutes at 4°C and the pellet suspended in 70% Ethanol (Absolute Stock, Fisher, UK) to sterilize the collagen. This solution was stirred at 4°C for 48 hr, and collected by centrifugation at 10,000 rpm for 10 min at 4°C. The collagen solution was then transferred in a laminar flow hood to sterile, pre-weighed 50 ml tubes. This was then frozen at -80°C overnight and lyophilised the following day using a freeze drier. The tubes were weighed and compared to their empty weight using a scale and the weight of the collagen determined. 0.15 M Acetic Acid was added to give a final concentration of collagen of 3 mg/ml.

### Traditional Acid Solubilisation - Whole Jellyfish Material

Separated jellyfish bells or tentacles were thawed overnight at 4°C and drained of any residual liquid. Weight was recorded for comparison to catch weight and final yield calculations. The samples were washed with distilled water to remove any foreign bodies until the bell or tentacles were almost clear. These were then blended roughly to produce a slush consistency of material ready for processing. The samples were then treated with 0.1M NaOH (1M Stock, Fisher, UK) at a sample/solution ratio of 1:10 (w/v). This was then stirred using a magnetic stirrer for 2 days at 4°C. The solution was then filtered through cheesecloth, followed by glass wool to remove any insoluble material. The solution was then poured into dialysis tubing and dialyzed against 5L of Dialysis Buffer at 4°C which was changed daily for 3 days. The collagen then began precipitating out of solution and appeared as a white solid. The collagen containing solution was then centrifuged at 10,000rpm / 16,000g for 10 minutes at 4°C using a centrifuge. This was then suspended in 500ml 0.15M acetic acid for 48h at 4°C to purify the collagen. 25g of solid NaCl was then added to give a concentration of 5% and overnight incubation at 4°C dissolved the salt, causing the displacement and precipitation of the collagen. The centrifugation, through to salting out was repeated twice to further purify collagen, followed by a final centrifugation at 10,000rpm for 10 minutes at 4°C. The final pellet was dissolved in 500mL 4°C 0.15 M Acetic Acid and dialyzed against Dialysis Buffer for 5 consecutive days at 4°C to remove all traces of NaCl, ensuring the Dialysis Buffer was changed daily. The solution was then centrifuged at 10,000rpm / 16,000g for 10 minutes at 4°C and the pellet suspended in 70% Ethanol (Absolute Stock, Fisher, UK) to sterilize the collagen. This solution was stirred at 4°C for 48 hr, and collected by centrifugation at 10,000 rpm for 10 min at 4°C. The collagen solution was then transferred in a laminar flow hood to sterile, pre-weighed 50 ml tubes. This was then frozen at -80°C overnight and lyophilised the following day using a freeze drier. The tubes were weighed and compared to their empty weight using a scale and the weight of the collagen determined. 0.15 M Acetic Acid was added to give a final concentration of collagen of 3 mg/ml.

### Membrane Extraction - Jellyfish

The frozen jellyfish material which had been earlier separated into either tentacles or bells were thawed at 4°C overnight and thoroughly washed with RO water, until no foreign bodies were present and the material was almost clear. This material was blended thoroughly until no chunks of material present were larger than 1mm in diameter. This was then pumped into the bioreactor vessel by use of a peristaltic pump and autoclaved tubing. The samples were then treated with 0.1M NaOH (1M Stock, Fisher, UK) at a sample/solution ratio of 1:10 (w/v). The solution was run under circulation loop 1 as demonstrated in figure 3 for 3 days to allow cellular matter and other non-collagenous material to be destroyed and, in particular, the hydrolysis of fats and proteins which can allow for their removal through the membrane system.

The solution was then dialysed using loop 2 as shown in figure 4 to remove NaOH from solution using a 10,000 NMWCO hollow fibre membrane. The reduction in volume is then replaced with Deionised water to maintain total volume of 20% reactor capacity. The solution was filtered until a pH <8 was obtained. The remaining capacity of the bioreactor was then filled to capacity with the appropriate volume of acid and RO water to make a final concentration of 0.5M acetic acid. This was then cycled under loop 3 as shown in figure 5 for 3 days to allow the collagen to interact and undergo partial cleavage of the telomeric regions.

Following this, the solution was again dialysed to remove the acid using loop 2 as shown in figure 4 using a 50,000 NMWCO hollow fibre membrane. The reduction in volume was replaced with water to maintain a total volume of 20% of reactor capacity and the solution was filtered until a pH >6.5 was obtained and subsequently replenished to 0.5M AcOH at 100L capacity to increase yield. This was repeated 3 times in total before the volume of solution was reduced as much as possible to leave a highly concentrated mixture ready to be dried. Once dialysed and concentrated, samples of the solution were lyophilised to determine dry weight of the collagen.

### Membrane Extraction - Bovine Insoluble Collagen

The bovine insoluble collagen was purchased from Sigma (UK) and stored at 4°C until use. This insoluble material was then hydrated in RO water and blended thoroughly until no chunks of material present were larger than 1mm in diameter. This was then pumped into the bioreactor vessel by use of a peristaltic pump and autoclaved tubing. The samples were then treated with 0.1M NaOH (1M Stock, Fisher, UK) at a sample/solution ratio of 1:10 (w/v). The solution was run under circulation loop 1 as demonstrated in figure 3 for 3 days to ensure non-collagenous material was destroyed as initial collagen purity was indicated at ~ 60%; this allowed for removal through the membrane system. The solution was then dialysed using loop 2 as shown in figure 4 to remove NaOH from solution using a 10 NMWCO membrane. The reduction in volume is then replaced with DI to maintain total volume of 20% reactor capacity. The remaining capacity of the bioreactor was then filled to capacity with the appropriate volume of acid and RO water to make a final concentration of 0.5M acetic acid. This was then cycled under loop 3 as shown in figure 5 for 3 days to allow the collagen to interact and undergo partial cleavage of the telomeric regions.

Following this, the solution was again dialysed, instead to remove the acid as in loop 2, and subsequently replenished to 0.5M AcOH at 100L capacity to increase yield. This was repeated 3 times in total before the volume of solution was reduced as much as possible to leave a highly concentrated mixture ready to be dried. Once dialysed and concentrated, samples of the solution were lyophilised to determine dry weight of the collagen. The overall process can be seen in figure 6.

### Single Alpha Chain Collagens (SACCs) Isolation - All species

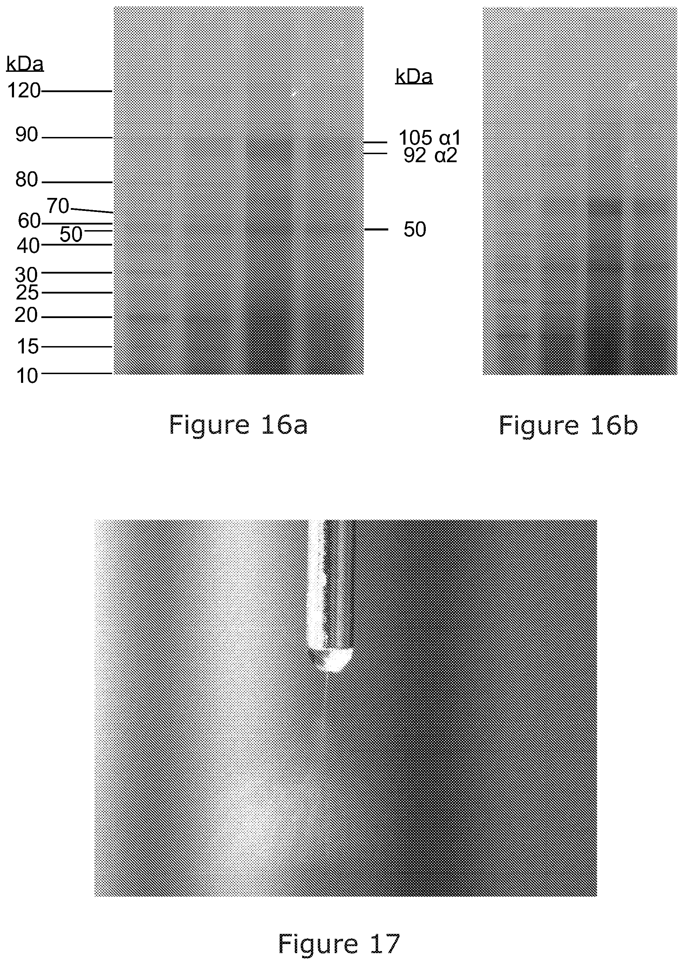

The collagen produced using the extraction protocols above was diluted to 100L at an AcOH concentration of 0.5M in preparation for SACCs. These materials were allowed to fully disperse overnight at 4°C while run under circulation loop 1 as shown in figure 3 then subjected to loop 4 as shown in figure 7 which comprised the inclusion of a 0.2µm hollow fibre membrane. The retentate was recycled and kept at 100L with input of fresh 0.5M AcOH using peristaltic pump and autoclaved tubing. The permeate of the system was collected aseptically into a 20L vessel which acted as a feed tank for loop 5 also shown in figure 7 which pumped the 0.2µm permeate solution across a 50 NMWCO hollow fibre membrane to concentrate the solution and allow the removal of the ~40kDa protein present in jellyfish collagen extracts seen in figure 16. The collagen solution was concentrated to <1L and collected aseptically by pumping from the sealed container for bottling in a class II biological cabinet which was cleaned under standard cell culture practice. The subsequent material containing either pure α1 & α2 collagen fibres or a mixture alpha and β-collagen chains was stored at 4°C for use in further characterisation or frozen at -80°C overnight and lyophilised and stored as a dry sponge/powder. The retentate from the 0.2µm filtration was concentrated to the minimum volume and stored for further characterisation.

### Collagen Characterisation

### SDS PAGE

SDS-PAGE analysis was carried out on samples before electrospinning to monitor for any changes to the presence or absence of particular chains of collagen alpha helix molecules. 7.5µL Collagen (3 ng/mL) dissolved in Acetic acid at 4°C was mixed with 2.5µL NuPAGE ^{®} LDS Sample Buffer (4X) (Life Technologies, UK). This mixture was then heated at 70°C in an incubator to reduce the collagen. Running buffer was prepared using 50mL 20X NuPAGE^{®} Tris-Acetate SDS Running Buffer added to 950mL deionized water. NuPAGE^{®} Tris Acetate Mini Gels were loaded into the holding clamp frame (Jencons, UK) and the running buffer added accordingly: 200mL inner chamber, 600mL outer chamber. The samples were then loaded alongside repeats of Benchmark^{™} Unstained Protein Ladder (Novex, UK) and the gel ran at 150V using a concord powerpack. Observed current ran from starting 45mA to final 27mA. After running, the gel was removed from the setup and placed into a tray for staining.

### Staining (Coomassie)

The gel was stained using Colloidal Blue staining kit (Invitrogen) based on work described by (Neuhoff, Arold, Taube, & Ehrhardt, 1988). Briefly, the gel was initially stained using a mixture of Stainer A, composed of ammonium sulphate in a phosphoric acid solution, and Stainer B containing the Coomassie Brilliant Blue (CBB) G-250 Stain, with further addition of ultra-pure water and methanol. This mixture forms particles of colloidal dye which in combination with the methanol, shifts the equilibrium of the CBB G-250 stain from the colloidal form to a molecularly dispersed dye, allowing complete diffusion into the dye. This solution is left to shake for a minimum of 3 hours and is then decanted and replaced with ultra-pure water overnight to remove any dye not bound to the protein bands. The stained gels were then imaged and examined using ImageJ software.

### Electrospinning

### Needle Based Electrospinning

Purified Collagen Extract was produced in house for use in these experiments from Bovine and Rhizostoma pulmo jellyfish sources. Collagen was prepared by examination using SDS PAGE and freeze drying sufficient material for use. To compare the impact of the presence of high molecular weight collagen chains on the ability of collagen to electrospin, isolation of α1 & α2 chains from the β and γ chains was carried out. Crossflow filtration was utilised with a hollow fibre membrane of 0.2µm nominal pore diameter. A 20L solution of 10mg/ml 'standard' collagen dissolved in 0.5M acetic acid (AcOH) was passed through the hollow fibre in a loop system at 1.5 Bar, 200L/h circulation. This allowed a weak solution of α1 & α2 single alpha chain collagens to be obtained, which was then concentrated to ~3mg/ml by utilising a 50,000 NMWCO hollow fibre membrane. This Process is shown in Figure 7. This was also examined using SDS PAGE analysis against the standard collagen extract, then lyophilised in preparation for electrospinning. To achieve adequate drying, samples were first aliquoted and frozen at -20°C for 24 hours, and then transferred to -80°C as Lyostat analysis of the collagen solutions showed an onset of collapse of -32.5°C (Carried out by Biopharma Technologies). The samples were transferred to the Scanvac CoolSafeTM freeze drier for drying. Once pressure was at 100µbar, shelf temperature was measured at -30°C and the primary drying stage of the process was carried out for 100 hours. Following this, shelf temperature was raised to +10°C for 15 Hours for the secondary drying stage. Samples were then removed from the drier and weighed before being stored in sealed containers with silica pouches at 4°C until use. A solution of 90% AcOH from Glacial in DI (v/v) was produced and chilled to 4°C. Following this, the dried collagen samples were weighed and added to an appropriate amount of AcOH to produce a collagen solution of between 10 - 50% (w/v). This was vortexed thoroughly until the collagen was fully dissolved and a consistent liquid was produced and was stored at 4°C to prevent the collagen from denaturing.

The electrospinning apparatus was set up using aluminium foil as the grounding target and was connected to ground. The syringe pump was set up to the correct diameter for a BD Plastipak^{®} 10ml needle and an 18G blunt tipped needle was used. These were rinsed with DI, followed by a blank 90% AcOH solution prior to the solution being taken into the syringe and any air was cleared before loading onto the syringe pump. The syringe pump was set to use a flow rate of 0.35mL per hour. The distance from needle tip to collector was 25cm and a voltage of 18kV was used. Voltage was applied when a droplet had formed at the tip of the needle and flow was maintained so volume of the droplet did not increase or reduce; an example of this setup is shown in figure 1. Taylor cone formation and electrospinning was observed using a camera and laser as shown in figure 17 and a 90% AcOH bath was placed in the electrospinning unit to prevent gelation of the droplet during periods of prolonged electrospinning. After electrospinning, the voltage was stopped and the equipment made safe, any remaining collagen was stored at 4°C for characterisation. The mat produced was then sampled and coated in a 5nm layer of chromium before being examined by scanning electron microscopy (SEM) using a Hitachi S4800 FEG-SEM at an acceleration voltage of 10kV and emission current of 9µA. Micrographs were taken with a magnification of 1500X for fibre diameter measurements, which were analysed using ImageJ software (Schneider, Rasband, & Eliceiri, 2012).

### Needle-less Electrospinning

For needle-less electrospinning, samples were freeze dried in the same manner and again dissolved at 25% concentration in a solution of 90% AcOH in DI. The solution was then placed in a bath and a mandrel with wires was submerged in the solution. The bath was inserted into the Nanospider NS Lab 200 System (Elmarco, Czech Republic) and a collection material placed over the grounding target. The Needle-less electrospinning process which has been previously described by (Burke et al., 2017); adapts the standard electrospinning approach to allow the formation several polymer jets simultaneously in order to allow for scalable fibre production. The process begins by applying a high voltage to a polymer bath which is continuously coating a 6 wire mandrel with droplets of polymer solution, which when exposed to the high voltage becomes elongated and Taylor cone formation occurs, forming multiple ejection points across the wire. The solution then undergoes rapid drying before being deposited on the collector. The material setup used a mandrel rotation speed at 10 revolutions per minute, a distance from mandrel tip to collector of 30cm and a mandrel to collector voltage of 80kV. Using these conditions, the current from mandrel to collector was observed to remain between 100 and 120µA, signalling consistent fibre formation to be occurring. The process was carried out for 1 hour before being removed and samples taken for SEM imaging and characterisation as described above. Any remaining collagen solution was removed and stored at 4°C for further solution testing, and the mat was sealed with silica packs to prevent excess moisture and kept at 4°C.

### PBS Electrospinning of Single Alpha Chain Collagens (SACCs)

SACCs which were lyophilised previously were removed from the fridge and dissolved at a concentration of 20% in a 1X PBS solution at 4°C. This was then mixed until fully dissolved and placed into a 5mL syringe. The solution was electrospun at a distance of 12cm and a voltage of 16kV with a flow rate of 0.35mL/hour. The resulting mat was taken for analysis by SEM, SDS PAGE and FTIR.

### Crosslinking

Collagen nanofiber mats were crosslinked for use in applications where stability in water or media was required at temperatures above 30°C. This was required due to i) the increased surface area gained by electrospinning causing swelling and movement of the fibres, ii) the temperature stability of jellyfish collagen being too low for use in tissue scaffolds and 3D cell culture experiments and iii) for single alpha chain collagens' stability in hydrophilic solutions such as PBS. In this process, the crosslinker selected was 1-ethyl-3-(3-dimethyl aminopropyl) carbodiimide (EDC).

### EDC Crosslinking

To crosslink the samples with EDC, a solution of 1% EDC in ethanol (w/v) was produced and samples were submerged in the crosslinking solution for a minimum of 4 hours while being clamped in place to prevent any swelling or shrinking deformation of the structure of the mat. Following this, the EDC solution was removed and the mats were soaked with pure ethanol for 24 hours, and then washed 3 times with DI before being placed in a desiccator to dry.

### FTIR of Collagen

Collagen samples were examined using a Perkin Elmer FTIR/ATR device using a soft tip. For liquid samples 5µL of solution was placed on the diamond stage and allowed to dry. The samples were then tested using a scan range from 4000 - 400 cm⁻¹. Once background correction was carried out, the data was normalised and was analysed using the Spectrum software and compared between samples to give a percentage similarity. This data was then exported and analysed for changes to secondary structure elements, as well as triple helix abundance.

### RESULTS

### Collagen Extraction

### Traditional Acid Solubilisation - Freeze Dried Jellyfish Material

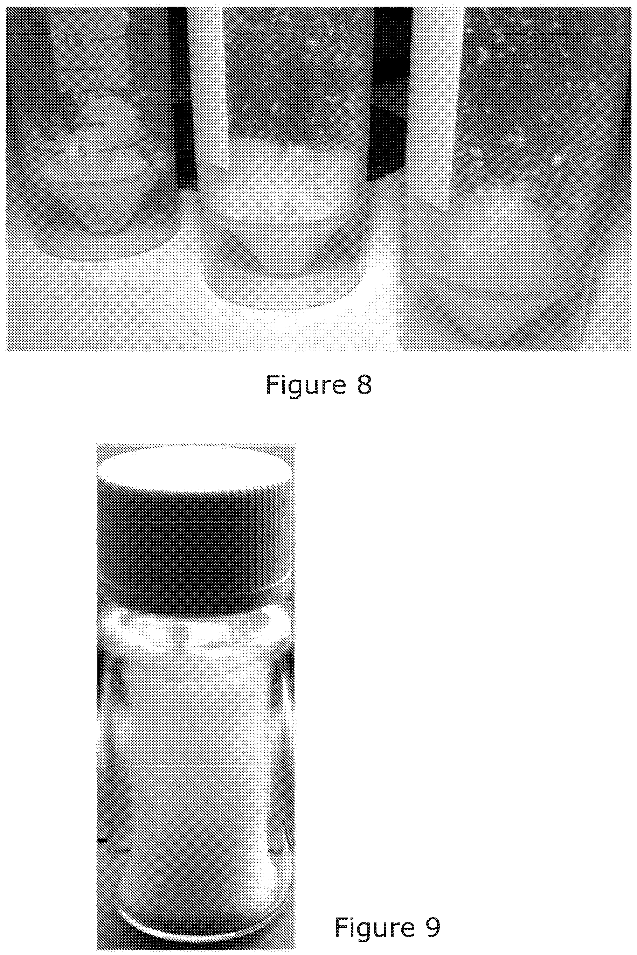

The freeze dried material which was extracted using the centrifugation based method achieved a yield of 0.061% based on the original catch weight of the jellyfish, signifying that 1kg of jellyfish would yield 0.61g of collagen. This yield makes the extraction of collagen very expensive and time consuming, and the collagen produced had a low structural rigidity, making its use for tissue engineering scaffolds ineffective. The process involved a two-week extraction protocol which was limited in effectiveness by the need for centrifugation, limiting the extractability to around 400mL per centrifugation cycle. A per person limit for this process is then described as 4L total volume of solution, equating to 400g of starting material, and just 0.24g of collagen per run as seen in figure 8. Electrospinning typically requires a minimum of 1mL of solution for a small scaffold to be produced, which based upon 10% collagen in the mixture would require 1 batch run of this process per 2 electrospun scaffolds and would require the use of HFP in order for the collagen to electrospin.

### Traditional Acid Solubilisation - Whole Jellyfish Material

The whole jellyfish material which was extracted using the centrifugation based method achieved a yield of 0.032% based on the original catch weight of the jellyfish, signifying that 1kg of jellyfish would yield 0.32g of collagen. This yield makes the extraction of collagen more time consuming, however the quality of the scaffold was slightly higher, retaining some sponge like appearance as seen in figure 9. The process involved the same two-week extraction protocol as above which was limited in effectiveness by the need for centrifugation, this means a per person limit for this process is just 0.13g of collagen per run. This would require one batch run per electrospun scaffold produced and would still require the use of HFP to electrospin the collagen into a nano-fibrous scaffold.

### Membrane Extraction - Jellyfish

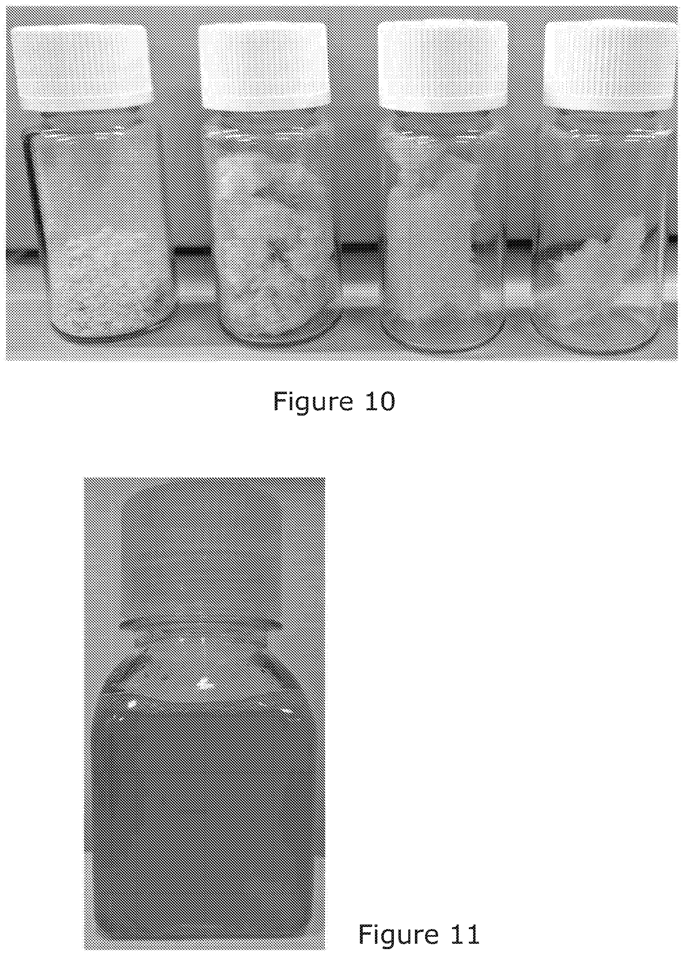

The membrane based method achieved a yield of 0.185% based on the original catch weight of the jellyfish, signifying that 1kg of jellyfish would yield 1.85g of collagen. This yield makes the extraction of collagen much less time consuming and the quality of the scaffold was much higher, with a very distinct sponge like appearance as seen in figure 10. The process involved a one-week extraction protocol due to the condensing of cellulose diafiltration stages into a single day each rather than a period of 3 days by utilising the hollow fibre membrane filtration system described. This was much more effective and removed the need for centrifugation, and because of the scalable nature of the process, a single person can run a plant of any size, further increasing output. The downside to this process is an excess of γ-chain collagen which causes a turbid solution as seen in figure 11. This process at a 100L scale as used in house produced 185g per one batch run which meant 1850 electrospun scaffolds could be produced. This process would however still require the use of HFP to electrospin the collagen into a nano-fibrous scaffold.

### Membrane Extraction - Bovine Insoluble Collagen

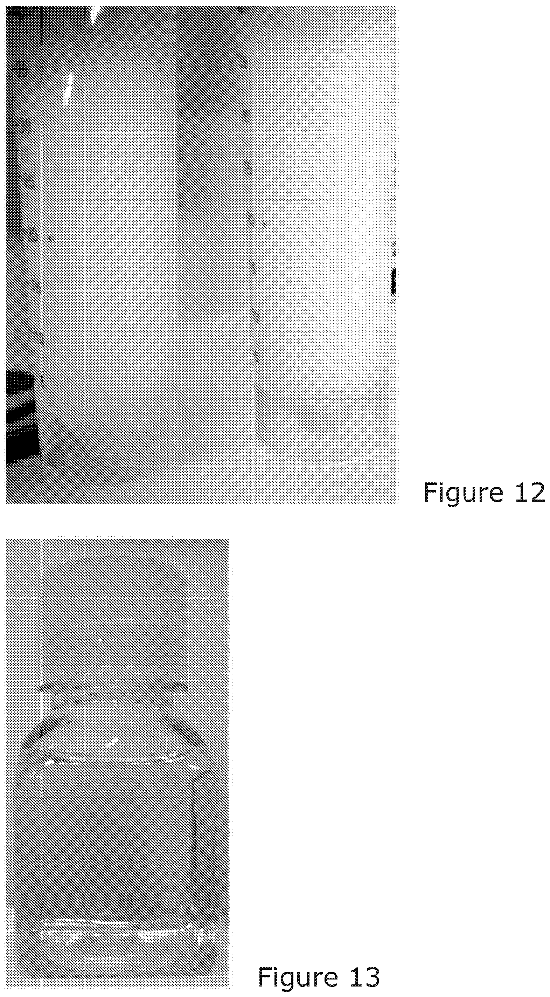

The insoluble bovine collagen which was extracted using the membrane extraction based method achieved a yield of 20% based on the original weight of the insoluble bovine material, signifying that 1kg of insoluble bovine collagen would yield 200g of soluble collagen. This yield makes the extraction of collagen much less time consuming and the quality of the scaffold was much higher, though the collagen appeared slightly more rigid in sponge form than the sponge like appearance of the jellyfish extract, however the bovine sponge had sufficient flexibility and was adequately compressible. The process involved a 5-day extraction protocol as above by utilising the hollow fibre membrane filtration system described without the need for NaOH treatment as this was carried out prior to material purchase. This was again much more effective by removing the need for centrifugation, and because of the scalable nature of the process, a single person can run a plant of any size, although this is dependent on the availability of insoluble collagen. This process at a 10L scale as used in house started with 400g which gave a total yield of 80g of soluble collagen per one batch run which meant 800 electrospun scaffolds could be produced. This process would however still require the use of HFP to electrospin the collagen into a nano-fibrous scaffold and produces a very turbid solution with a large quantity of γ-chains as seen in figure 12.

### Single Alpha Chain Collagens (SACCs) Isolation - All species

The collagen monomers produced using this method showed good solubility, clarity and low viscosity at concentrations up to 10mg/mL. When re-dissolved for electrospinning, the solution was a deep brown shade which was viscous enough to hold a droplet at the end of an 18G needle however was noticeably lower than the same concentration of standard extract and was noticeably less opaque, suggesting increased solubility in the acetic acid-water mixture. The concentration was raised from 10% collagen to 25% to prevent spitting of single alpha chain collagen solution as was the case at 10% which was used previously.

For jellyfish SACCs isolation, the extraction used the 0.2µm hollow fibre filtration and 50,000 NMWCO based method and achieved a yield of 12.5% based on the separation from the soluble collagen mixture, signifying that 1kg of soluble bovine collagen would yield 125g of SACCs. This yield makes the extraction of SACCs less efficient than that of the standard membrane extraction process, and the process involved a 10-day extraction protocol due to the added time of utilising the extra 0.2µm and 50 000 NMCO hollow fibre membrane filtration steps described. This allowed for the effective removal of β and γ collagen chains and due to the nature of the process, a single person can run a plant of any size, further increasing output. This process at a 100L scale as used in house produced 185g of standard collagen which was processed to produce 23.1g of SACCs per one batch run which meant 92 electrospun scaffolds could be produced. This process has the added benefit of not requiring the use of HFP to electrospin the collagen into nano-fibrous scaffolds, but instead can utilise a benign solvent such as acetic acid or a physiological buffer such as PBS. The solution produced was of very good clarity due to the absence of γ-chains as seen in figure 13 and produces a pure white powdered scaffold when lyophilised as seen in figure14.

For bovine SACCs isolation, the SACCs which were extracted using the 0.2µm hollow fibre filtration and 50,000 NMWCO based method achieved a yield of 10% based on the separation from the soluble collagen mixture, signifying that 1kg of soluble bovine collagen would yield 100g of SACCs. This yield makes the extraction of bovine SACCs less efficient than that of the standard membrane extraction process, and the process involved an 8-day extraction protocol due to the added time of utilising the extra 0.2µm and 50 000 NMCO hollow fibre membrane filtration steps described. This allowed for the effective removal of β and γ collagen chains and due to the nature of the process, a single person can run a plant of any size, further increasing output, again dependent on the availability of insoluble collagen precursor. This process at a 10L scale as used in house produced 80g of standard collagen which was processed to produce 8g of SACCs per one batch run which meant 32 electrospun scaffolds could be produced. This process and the use of the SACCs has the added benefit of not requiring the use of HFP to electrospin the collagen into nano-fibrous scaffolds, but instead can utilise a benign solvent such as acetic acid or a physiological buffer such as PBS.

### SDS PAGE

SDS PAGE analysis of the collagen samples showed the distinctive collagen α1 & α2 bands at ~105 and ~92kDa respectively, β chain expression as well as the presence of γ chains. These are shown in lanes 7-10 of figure 15.

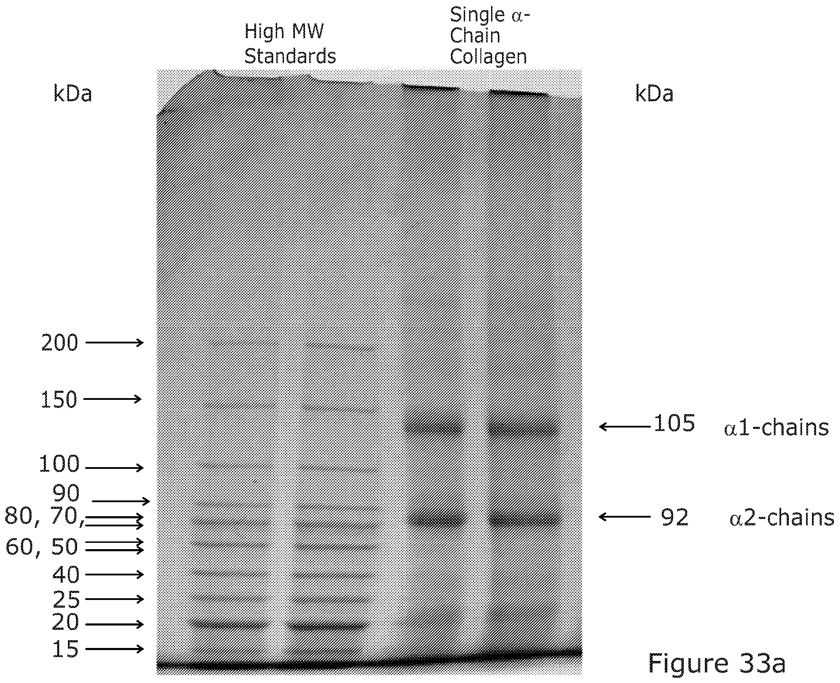

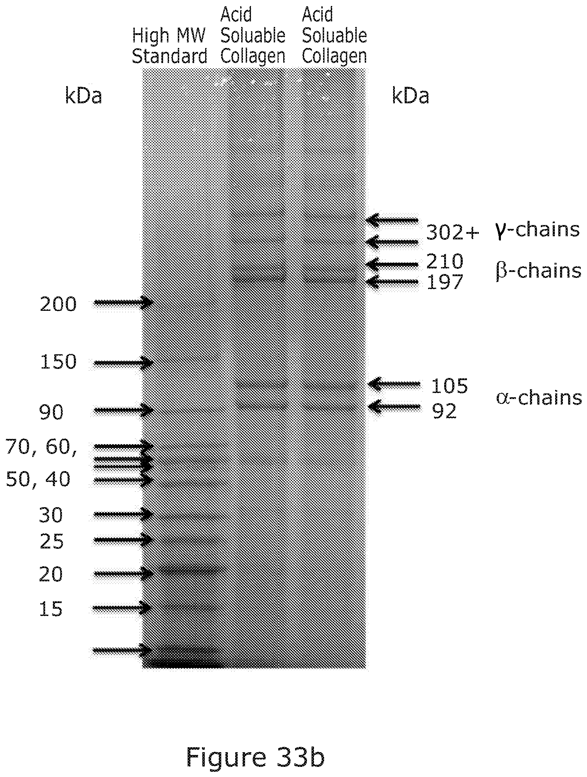

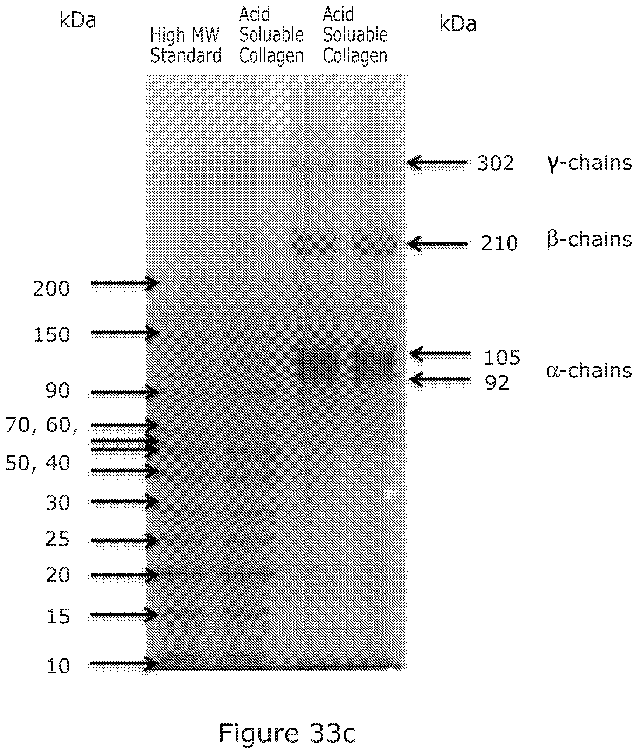

The jellyfish derived collagen that was examined using SDS PAGE showed the distinct α1 & α2 chains associated with acid solubilised collagen, as well as β and γ chains, which indicates the presence of both acid soluble collagen α1 & α2 chains, as well as a portion of semi-soluble collagen in solution which was not cleaved by digestion or removed when samples were centrifuged prior to testing for SDS PAGE. In order to isolate the α1 & α2 chains from the β and γ chains, crossflow filtration was utilised with a hollow fibre membrane of 0.2µm nominal pore diameter. Once separation of sufficient quantity of α1 & α2 single alpha chain collagens was obtained and concentrated using a 50,000 NMWCO hollow fibre membrane, this was also examined using SDS PAGE analysis, as seen in figure 16a and figure 33a. This was then freeze dried in preparation for electrospinning. This showed distinct banding at ~ 92 and 105 kDa representing separated monomers of α1 & α2 collagens without the high abundance of β & γ chain collagens. This demonstrates the effective removal of high molecular weight collagens as desired while retaining α-chains used for electrospinning. The SDS PAGE shows a band present at approximately 40kDa which was not removed in this instance due to insufficient cycles through the 50,000 NMWCO membrane cycle. This can be removed with added cycles of fresh AcOH into the solution and also aids in the removal of other background substances which are causing a haze to appear at the bottom of the gel. The collagen that was electrospun was re-dissolved in 0.5M AcOH and tested; this can be seen in figure 16b. In contrast, however, standard acid soluble collagen comprises a mixed extract of alpha chain collagen but also contaminating higher molecular weight beta and gamma chains, which limits downstream utility (figures 15 and 33b-c)

### Electrospinning

### Jellyfish single alpha chain collagens (SACCs) Electrospinning

Collagen was freeze dried using a primary drying shelf temperature of -30°C for 70h. Secondary drying was carried out with a shelf temperature of +20°C for 15h. Samples removed and placed in sealed containers showed that residual moisture for all experiments was between 2 - 5 %.

The jellyfish SACCs were optimised as follows: 25% Collagen / AcOH solution electrospun at 18kV, 20cm and 0.35ml/hour produced a constant droplet with a single taylor cone as shown in figure 17.

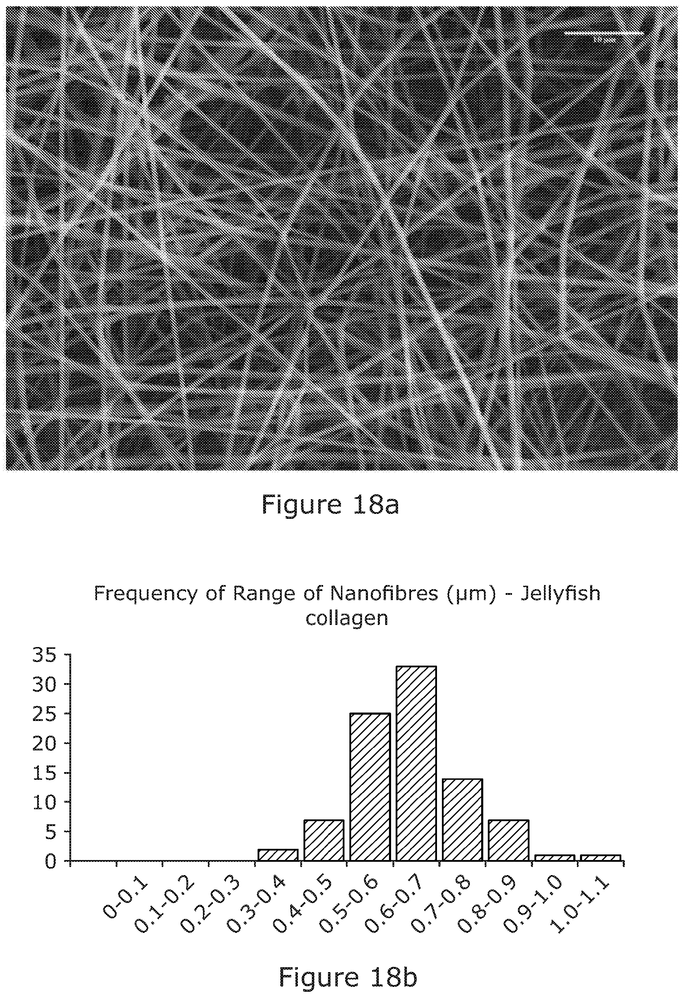

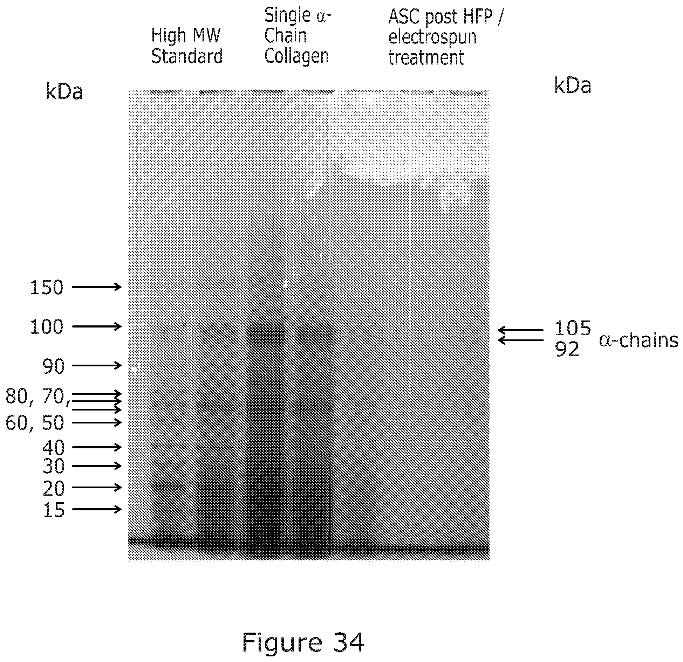

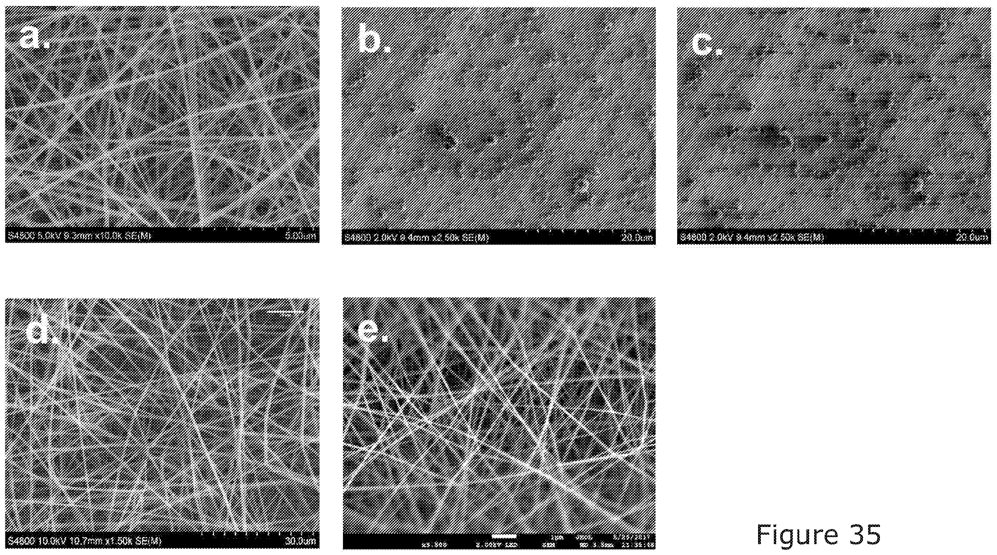

The samples were examined under SEM and showed a mean fibre diameter of 646nm with a standard deviation of 121nm based on 90 fibre measurements. Micrographs of the needle electrospun fibres can be seen in figure 18a with frequency of range seen in figure 18b and figure 35d. In contrast, acid soluble collagen can only be electrospun in harsh denaturing solvents such as hexafluoropropanol (HFP) (figure 35a) and when electrospun in benign solvents such as acetic acid (figure 35b-c), in contrast to SACC, produces nanospheres due to the presence of high molecular weight beta and gamma chains. To this extent, electrospinning in harsher solvents destroys the chain structure and thus the ability of SACC to spin in benign solutions retains chain structure promoting its utility (figure 34).

### Bovine single alpha chain collagens (SACCs) Electrospinning

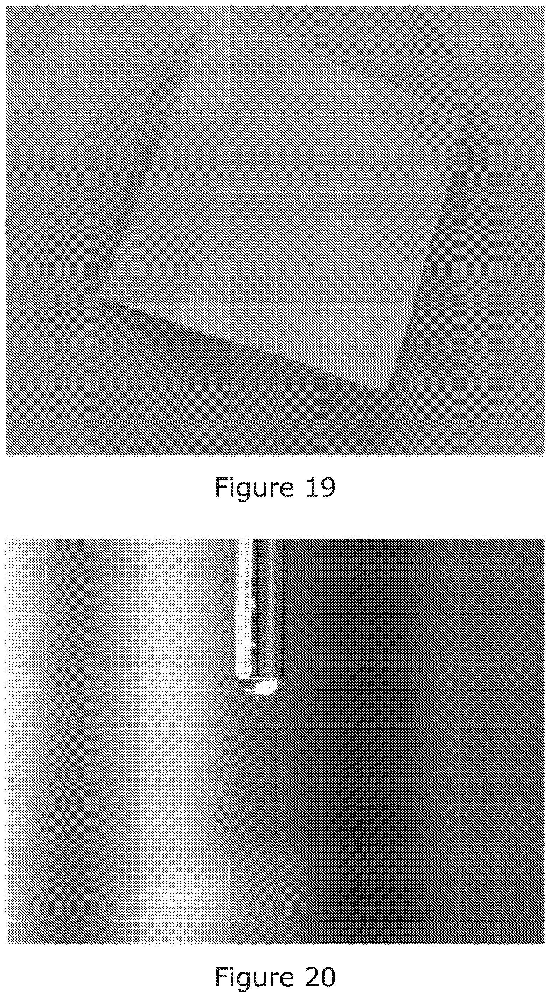

The bovine SACCs were optimised as follows: 35% Collagen / AcOH solution electrospun at 20kV, 18cm and 0.35ml/hour produced a constant droplet with a single taylor cone as shown in figure 20.

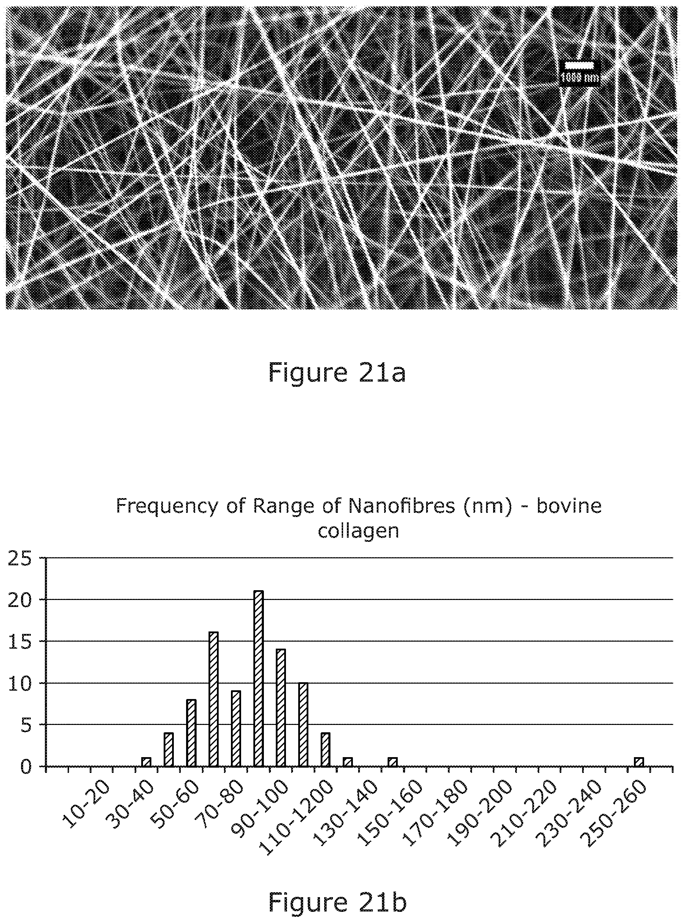

The samples were examined under SEM and showed a mean fibre diameter of 646nm with a standard deviation of 121nm based on 90 fibre measurements. Micrographs of the needle electrospun fibres can be seen in figure 21a and the frequency of range can be seen in figure 21b. Thus SACC can be obtained from multiple species as a source and retains the same properties.

### Needle-less Electrospinning

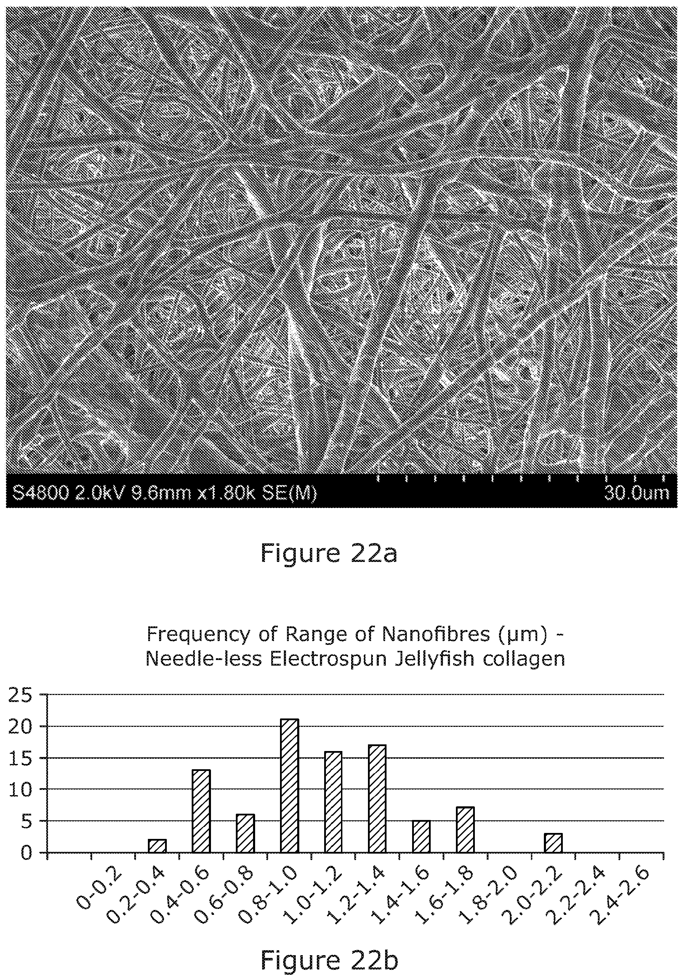

Collagen was freeze dried using a primary drying shelf temperature of -30°C for 70h. Secondary drying was carried out with a shelf temperature of +20°C for 15h. Samples removed and placed in sealed containers showed that residual moisture for all experiments was between 2 - 5 %. The jellyfish SACCs were optimised as follows: 25% Collagen / AcOH solution (90% AcOH in DI water) electrospun at 60kV, 20cm and 10 rotations / minute produced a collagen mat at a consistent rate. The samples were examined under SEM and showed a mean fibre diameter of 1128nm with a standard deviation of 406nm based on 90 fibre measurements. Micrographs of the needle-less electrospun fibres can be seen in figure 22a with frequency of range seen in figure 22b.

### PBS Electrospinning of SACCs

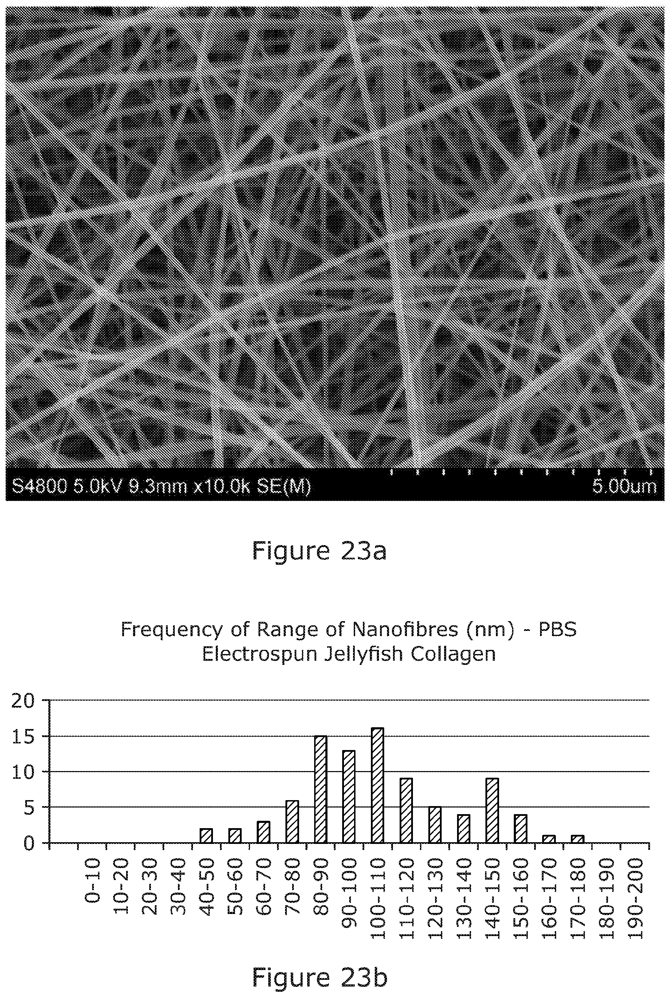

The SACCs which were dissolved in a standard PBS solution were more opaque than the acetic acid solution used above and appeared slightly more viscous. The concentration was optimised at 20% SACCs which gave a good fibre production without spitting of solution. Samples of collagen which were electrospun from a PBS formulation were examined under SEM and fibre diameter was shown to be 105nm ± 28nm as shown in figure 23. SEM images can also be seen in figure 35e.

### Crosslinking

### EDC Crosslinking

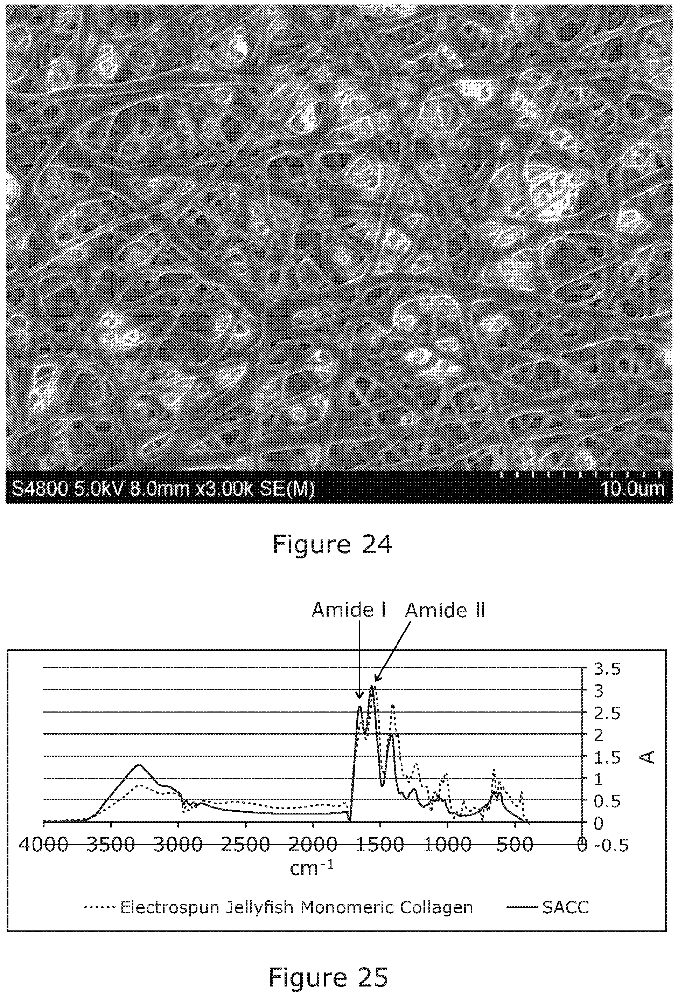

Crosslinking of collagen fibres using a 1% EDC solution using our compression method yielded mats with little change from the original on the macro scale, SEM imaging was taken of the processed fibres as seen in figure 24 and fibre diameter was 125, with a standard deviation of 32nm.

### FTIR

Collagen samples were examined using a Perkin Elmer FTIR/ATR device using a soft tip. A small volume of solution was placed on the diamond stage and allowed to dry. The samples were then tested using a scan range from 4000 - 400 cm⁻¹. Once background correction was carried out, the data was normalised to 3 Absorbance units (A) at the average amide II peak of 1558cm⁻¹ and was analysed using the Spectrum software which compared between samples to give a percentage similarity.

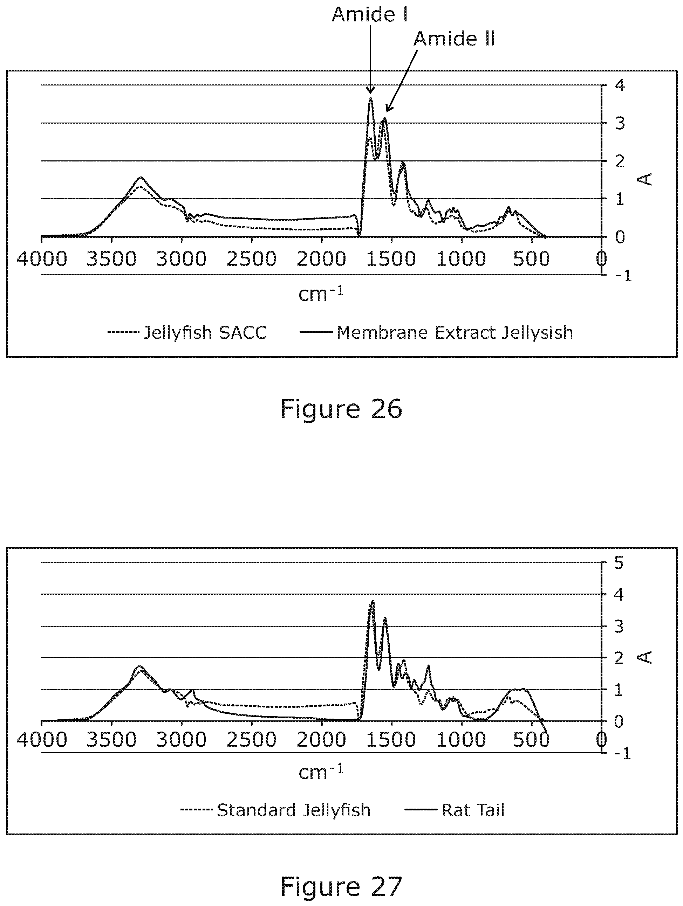

The Findings of this work showed that by producing SACCs it was possible to obtain pure α1 & α2 chains which are not found to be naturally occurring. Their enhanced solubility raised questions as to whether the proteins had been partially denatured, However, FTIR examination of vibrations relating to secondary structure has demonstrated that the only significant change is a reduction in the amide 1 region due to the absence of triple helix (γ-chain collagens). This in turn causes a change in the ratio of amide 1 and amide 2, with amide 2 becoming the more pronounced peak, as seen in figures 25 and 26.

Collagen samples from base materials were tested against their electrospun counterparts and the percentage homology was examined. This data was also compared to rat tail type I collagen (Sigma, UK) to act as an external standard. The SACCs differed from standard collagen by a reduction in the triple helix absorption at 1638cm⁻¹. The peaks of interest which were examined are listed in table 1. The comparisons of collagen in preceding and electrospun forms are shown in figure 22b All data was baseline corrected and normalised using Perkin Elmer Spectrum software at 1558cm⁻¹.

The region representing the relative abundance of triple helix was reduced in SACCs samples and electrospun samples as expected due to the reduction in y-chain abundance in the samples. Uncoiled, alpha helix, beta sheet secondary structured did not alter in ratios of abundance when the influence of the reduction of triple helix was accounted for; suggesting the secondary structure of the alpha chains has not been altered from the secondary structure of standard collagen samples as shown in figure 26.

### Collagen Amino Acid Chain Modelling

Collagen solubility is an area which has been debated thoroughly in the literature; particularly ascertaining to electrospun scaffolds. We have demonstrated that SACCs are soluble in physiological buffers and benign solvents such as AcOH. To gain a better understanding of this increased solubility we examined the structure of collagen and the derivatives thereof.

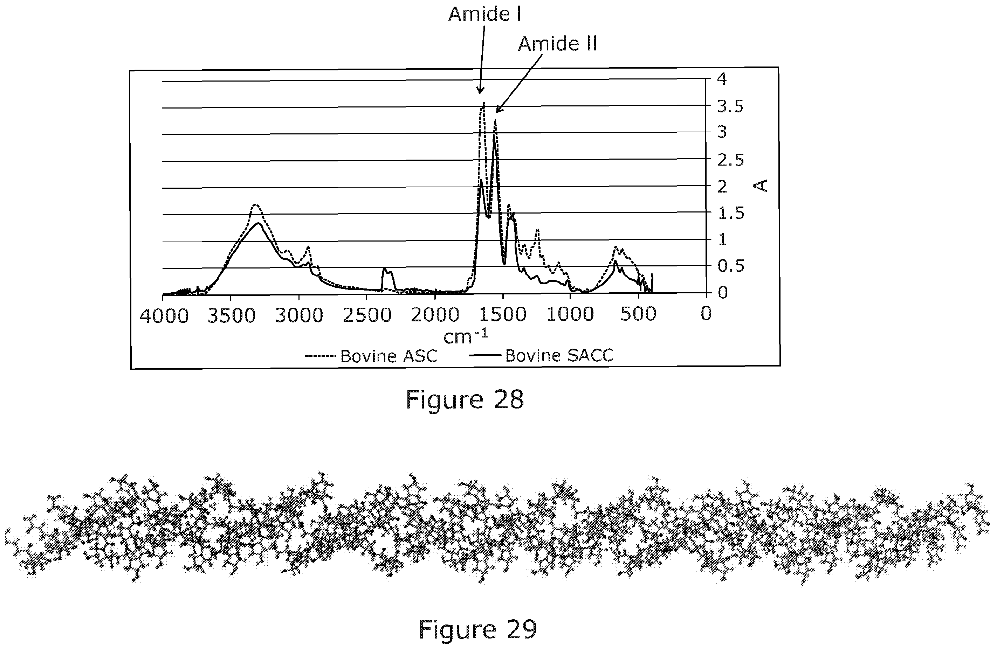

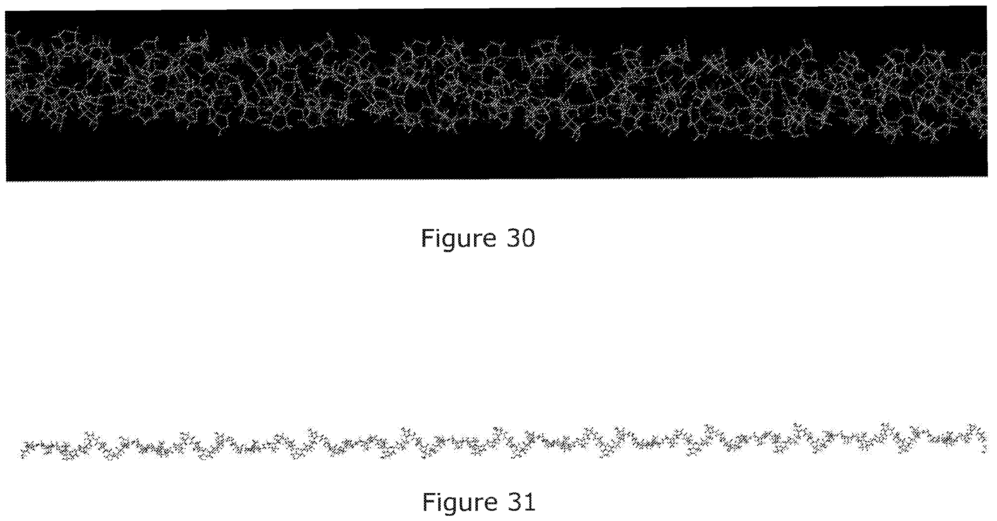

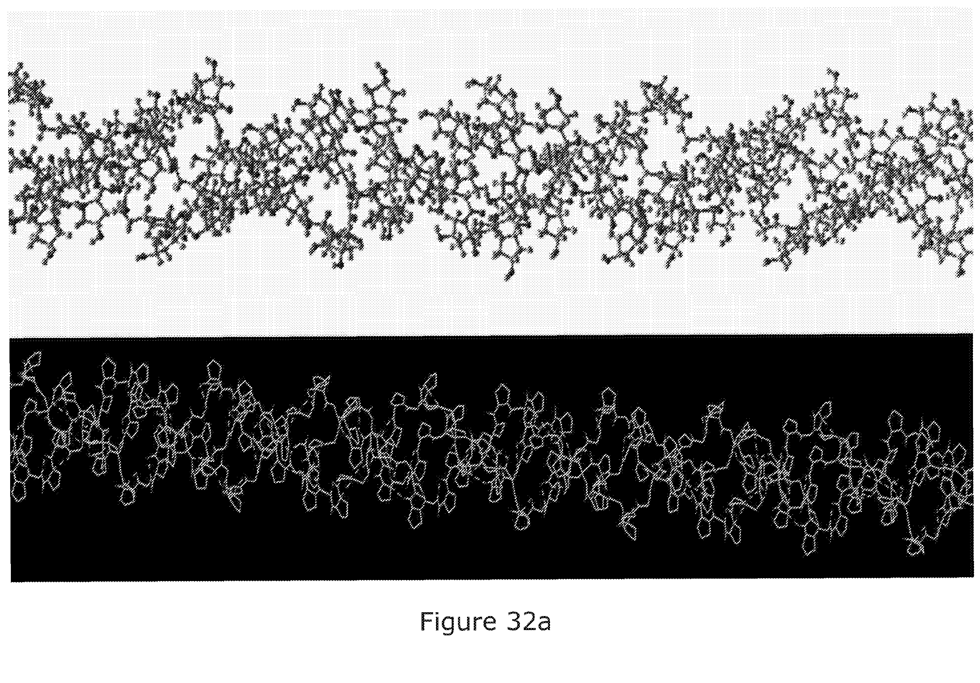

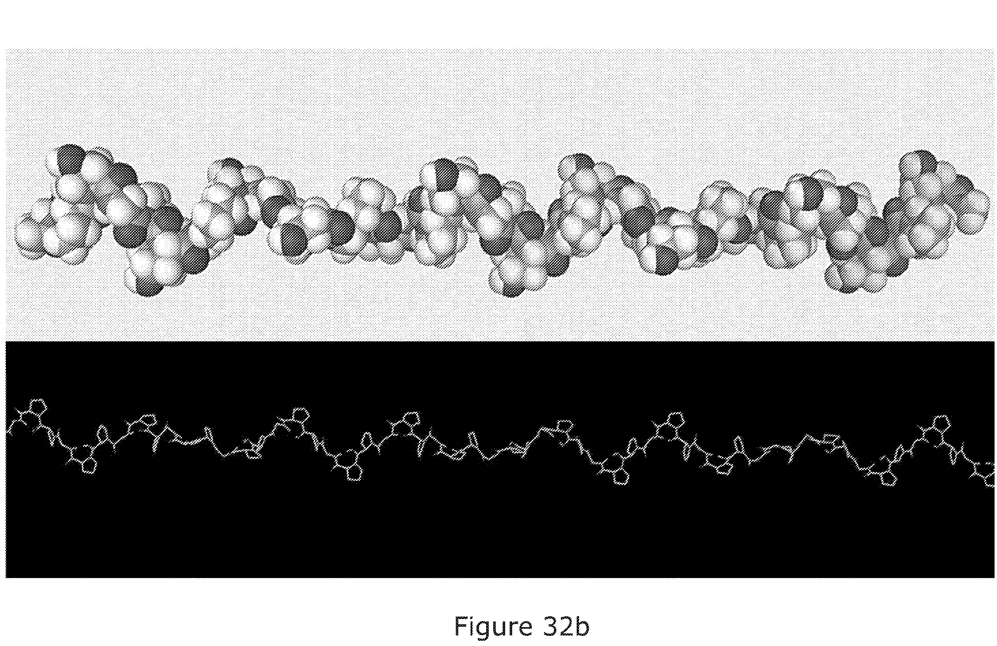

In order to assess why collagen in non-triple helix formation is able to dissolve readily we modelled the structure of collagen chains using the Abalone software to build a model protein sequence consisting of the Gly-X-Y repeat sequence; where X was Proline and Y was Hydroxyproline. This model was arranged with a hybrid Lui Storey and Conjugate Descent optimisation (LS-CD) as seen in figure 29 which was then exported into Zeus software and hydrogen bonding was examined between chains as seen in figure 30. The triple helical formation showed hydrogen bonding where possible occurred on the inward facing functional groups of amino acids which helped to stabilise the molecule. When the α-chains were separated from the triple helix molecule the SACCs, as shown in figure 31, had an abundance of hydrogen bonding sites which allow for increased solubility. This is further shown and modelled in figure 32. Based on this modelling, ASC has approximately 90 free hydrogen bonds per chain in contrast to approximately 530 free hydrogen bonds per chain of SACC.

This gives good evidence as to why the SACCs is both soluble and able to undergo electrospinning, with chains able to gain affinity to both the solvent and themselves during the electrospinning process.

### SUMMARY

We have shown that by using a multi-step membrane separation process it is possible to produce SACCs and so obtain pure α1 &/or α2 chains, which are not found to be naturally occurring. These SACCs composed of α1 &/or α2 chains can be separated from the acid solubilised collagen mixture successfully giving rise to new opportunities for the use of this physiologically soluble collagen in applications such as wound aerosol sprays, which were not previously possible because of the insoluble nature of collagen in a suitable solvent system.

Further, by using less harsh isolation techniques, possible due to the alternative separation methodology, SACCs maintain their secondary structure and can be subsequently electrospun to produce nanofibrous mats which mimic native ECM conditions without the need to include a co-polymer component to allow electrospinning to occur. This solves the unmet need for collagen that can be formulated into physiological systems for applications ranging from injectables, implants, bio-active wound dressings as well as a suitable tissue engineering substrate. The creation of this unique polymer can give new life to the field of biomimetic scaffold production by introducing a pathway for its own use across different fields.

We have also demonstrated that collagen which contains the high molecular weight chains conforms to traditional use of HFP in collagen electrospinning in both mammalian and non-mammalian systems but demonstrates the time sensitivity of the solution produced. This therefore presents a promising finding for collagen electrospinning which allows the collagen formulation to be stored prior to electrospinning and can minimise wastage from scaffold production for an expensive product which is in great need. We have shown that the SACCs formulation works in a variety of organisms and show cross-species homology in both the production and electrospinning of the single alpha chain collagen chains.

**Table 1.**

| Expected | Corresponding Vibration |
|---|---|
| λ (±2cm⁻¹) | |
| 1690 | Parallel β-Sheets |
| 1680 | Anti-Parallel β-Sheets |
| 1668 | B-Turns |
| 1658 | A-Helix |
| 1647 | Unordered |
| 1638 | Triple Helix |
| 1625 | Parallel β-Sheets |
| 1612 | B-Turns |

**Table 2.**

| **Collagen Type** | **Approximate Length (Number of amino acids)** | | |
|---|---|---|---|
| | α1 | α2 | α3 |
| 1 | 1464 | 1366 | |
| 2 | 1487 | | |
| 3 | 1466 | | |
| 5 | 1838 | 1499 | 1745 |
| 24 | 1714 | | |
| 26 | 3051 | | |

### References

Burke, L., Mortimer, C. J., Curtis, D. J., Lewis, A. R., Williams, R., Hawkins, K., ... Wright, C. J. (2017). In-situ synthesis of magnetic iron-oxide nanoparticle-nanofibre composites using electrospinning. Materials Science and Engineering C, 70, 512-519. https://doi.org/10.1016/j.msec.2016.09.014
Neuhoff, V., Arold, N., Taube, D., & Ehrhardt, W. (1988). Improved staining of proteins in polyacrylamide gels including isoelectric focusing gels with clear background at nanogram sensitivity using Coomassie Brilliant Blue G250 and R250. ELECTROPHORESIS, 9(6), 255-262. https://doi.org/10.1002/elps. 1150090603
Schneider, C. A., Rasband, W. S., & Eliceiri, K. W. (2012). NIH Image to ImageJ: 25 years of image analysis. Nat Meth, 9(7), 671-675. Retrieved from http://dx.doi.org/10.1038/nmeth.2089
Brodsky B., Ramshaw J. A. (1997) The collagen triple-helix structure. Matrix Biol. 15, 545-554 CrossRefMedlineGoogle Scholar
Kielty C. M., Grant M. E. (2002) The Collagen family: structure, assembly and organization in the extracellular matrix. In Connective Tissue and Its Heritable Disorders: Molecular, Genetic, and Medical Aspects ( Royce P. M., Steinmann B. , eds) 2nd Ed., pp. 158-222, Wiley-Liss, New York Google Scholar
Veit G., Kobbe B., Keene D. R., Paulsson M., Koch M., Wagener R. (2006) Collagen XXVIII, a novel von Willebrand Factor A domain-containing protein with many imperfections in the collagenous domain. J. Biol. Chem. 281, 3494-3504 Abstract/FREE Full Text
Shoulders M. D., Raines R. T. (2009) Collagen structure and stability. Annu. Rev. Biochem. 78, 929-958 CrossRefMedlineGoogle Scholar

## Claims

1. Isolated single alpha chain collagens (SACCs) in aqueous solution wherein said SACCs is or has:
a) a single chain polypeptide existing as an alpha helix and having a molecular weight of 80-120kDa;
b) a repeating sub structure of Glycine-X-Y (where X can be any amino acid, and Y is proline or hydroxyproline);
c) non-helical N-terminal and/or C-terminal ends; and further wherein
d) said aqueous solution i) does not include beta or gamma collagen, ii) contains an amount of SACCs per total collagen protein content of at least 75% and iii) comprises SACCs at a concentration of at least 85% w /v.

2. The isolated SACCs according to claim 1 wherein said aqueous solution contains an amount of SACCs per total collagen protein content selected from the group comprising: at least 85%, 86%, 87%, 88%, 89%, 90%, 91%, 92%, 93%, 94%, 95%, 96%, 97%, 98%, 99% or 100%.

3. The isolated SACCs according to any one of claims 1-2 wherein said SACCs are of mammalian or cnidarian origin.

4. The isolated SACCs according to claim 3 wherein said SACCs are of bovine porcine, equine, or jellyfish origin.

5. The isolated SACCs according to any one of claims 1-4 wherein said SACCs are recombinant or of recombinant origin.

6. The isolated SACCs according to any one of the preceding claims wherein each SACC comprises approximately 1000-3500 amino acids.

7. The isolated SACCs according to claim 6 wherein each SACC comprises approximately 1000-1500 amino acids.

8. The isolated SACCs according to any one of claim 1-7 for use in electrospinning.

9. An electrospinning solution comprising the isolated SACCs according to any one of claims 1-7 and a solution suitable for electrospinning.

10. A method for the production or purification of single alpha chain collagen (SACC) comprising:
a) obtaining an extraction of collagen in alkaline solution;
b) filtering the extraction using at least one first filter membrane having a Nominal Molecular Weight Cut-Off (NMWCO) in the range of 1 - 100,000 to produce a first filter retentate;
c) adjusting the pH of the first filter retentate to have an acidic pH of less than 3 thereby at least partially solubilising the collagen bound by same;
d) filtering the at least partially solubilised first filter retentate until a pH of greater than 6 is achieved using said at least one first filter membrane having a Nominal Molecular Weight Cut-Off (NMWCO) in the range of 1 - 100,000 to produce a second filter retentate;
e) adjusting the pH of the second filter retentate to have a pH of less than 3 thereby solubilising the collagen bound by same;
f) filtering the second filter retentate using at least one second filter membrane wherein the second filter membrane is a micro-porous membrane comprising a pore size between about 0.05 to 2um and every 0.01µm integer therebetween to produce a first filtrate comprising SACCs; and
g) optionally, filtering the first filtrate using said at least one first filter membrane to produce a second second retentate comprising single alpha chain collagen.

11. The method according to claim 10 wherein the first filter membrane has a NMWCO selected from 10,000 or 50,000.

12. The method according to any one of claims 10 to 11 wherein in step a) the original collagen containing sample is treated with alkaline or basic solution to allow cellular matter and other non-collagenous material to be destroyed.

13. The method according to any of claims 10 to 12 wherein in step b) the extraction is filtered until a pH of less than 8 is achieved and a first filter retentate is obtained.

14. The method according to any one of claims 10 to 13 wherein in step b) the first filter retentate is supplemented, before during or after filtering, with deionised water and/or in step d) the second filter retentate is supplemented with deionised water.

15. The method according to any one of claims 10 to 14 wherein steps c) - d) are repeated at least once to improve yield and purity.

16. The method according to any one of claims 10 to 15 wherein steps c) - d) are performed in a closed circulatory system that either runs constantly or runs constantly during the performance of the said specified steps.

17. The method according to any one of claims 10 to 16 wherein said filtering involves the process of dialysis.

18. The method according to any one of claims 10 to 17 wherein in step f) and/or g) the second filter retentate or the first filtrate is/are filtered until a pH of less than 8 is achieved.

19. The method according to any one of claims 10 to 18 wherein said collagen is extracted from mammalian or cnidarian tissue.

## Patentansprüche

1. Isolierte Einzel-Alpha-Ketten-Kollagene (SACCs) in wässriger Lösung, wobei die SACCs Folgendes sind oder aufweisen:
a) ein einkettiges Polypeptid, das als eine Alpha-Helix vorliegt und ein Molekulargewicht von 80-120 kDa aufweist;
b) eine sich wiederholende Unterstruktur von Glycin-X-Y (wobei X eine beliebige Aminosäure sein kann und Y Prolin oder Hydroxyprolin ist);
c) nicht helikale N-terminale und/oder C-terminale Enden; und wobei ferner
d) die wässrige Lösung i) kein Beta- oder Gamma-Kollagen beinhaltet, ii) eine Menge an SACCs pro Gesamtkollagenproteingehalt von mindestens 75 % enthält und iii) SACCs in einer Konzentration von mindestens 85 Gew.-/Vol.-% umfasst.

2. Isolierte SACCs nach Anspruch 1, wobei die wässrige Lösung eine Menge an SACCs pro Gesamtkollagenproteingehalt enthält, die aus der Gruppe ausgewählt ist, die Folgendes umfasst: mindestens 85 %, 86 %, 87 %, 88 %, 89 %, 90 %, 91 %, 92 %, 93 %, 94 %, 95 %, 96 %, 97 %, 98 %, 99 % oder 100 %.

3. Isolierte SACCs nach einem der Ansprüche 1-2, wobei die SACCs von Säugetieren oder Cnidaria stammen.

4. Isolierte SACCs nach Anspruch 3, wobei die SACCs von Rindern, Schweinen, Pferden oder Quallen stammen.

5. Isolierte SACCs nach einem der Ansprüche 1-4, wobei die SACCs rekombinant sind oder rekombinanten Ursprungs sind.

6. Isolierte SACCs nach einem der vorhergehenden Ansprüche, wobei jedes SACC etwa 1000-3500 Aminosäuren umfasst.

7. Isolierte SACCs nach Anspruch 6, wobei jedes SACC etwa 1000-1500 Aminosäuren umfasst.

8. Isolierte SACCs nach einem der Ansprüche 1-7 zur Verwendung beim Elektrospinnen.

9. Elektrospinnlösung, umfassend die isolierten SACCs nach einem der Ansprüche 1-7 und eine für Elektrospinnen geeignete Lösung.

10. Verfahren für die Herstellung oder Reinigung von Einzel-Alpha-Ketten-Kollagen (SACC), umfassend:
a) Erlangen einer Extraktion von Kollagen in alkalischer Lösung;
b) Filtern der Extraktion unter Verwendung mindestens einer ersten Filtermembran, die eine nominale Molekulargewichtsgrenze (NMWCO) im Bereich von 1 - 100.000 aufweist, um ein erstes Filterretentat zu erzeugen;
c) Einstellen des pH-Werts des ersten Filterretentats, sodass es einen sauren pH von weniger als 3 aufweist, wodurch das durch diesen gebundene Kollagen mindestens teilweise löslich gemacht wird;
d) Filtern des mindestens teilweise löslich gemachten ersten Filterretentats, bis ein pH-Wert von mehr als 6 erreicht ist, unter Verwendung der mindestens einen ersten Filtermembran, die eine nominale Molekulargewichtsgrenze (NMWCO) im Bereich von 1 - 100.000 aufweist, um ein zweites Filterretentat zu erzeugen;
e) Einstellen des pH-Wertes des zweiten Filterretentats, sodass es einen pH-Wert von weniger als 3 aufweist, wodurch das durch diesen gebundene Kollagen löslich gemacht wird;
f) Filtern des zweiten Filterretentats unter Verwendung mindestens einer zweiten Filtermembran, wobei die zweite Filtermembran eine mikroporöse Membran ist, die eine Porengröße zwischen etwa 0,05 bis 2 µm und jeden Schritt um 0,01 µm dazwischen umfasst, um ein erstes Filtrat zu erzeugen, das SACCs umfasst; und
g) optional Filtern des ersten Filtrats unter Verwendung der mindestens einen ersten Filtermembran, um ein zweites zweites Retentat zu erzeugen, das Einzel-Alpha-Ketten-Kollagen umfasst.

11. Verfahren nach Anspruch 10, wobei die erste Filtermembran einen NMWCO aufweist, der aus 10.000 oder 50.000 ausgewählt ist.

12. Verfahren nach einem der Ansprüche 10 bis 11, wobei in Schritt a) die ursprüngliche kollagenhaltige Probe mit alkalischer oder basischer Lösung behandelt wird, um die Zerstörung von Zellmaterial und anderem nicht kollagenen Material zu ermöglichen.

13. Verfahren nach einem der Ansprüche 10 bis 12, wobei in Schritt b) die Extraktion filtriert wird, bis ein pH-Wert von weniger als 8 erreicht ist und ein erstes Filterretentat erhalten wird.

14. Verfahren nach einem der Ansprüche 10 bis 13, wobei in Schritt b) das erste Filterretentat vor, während oder nach dem Filtern mit entionisiertem Wasser ergänzt wird und/oder in Schritt d) das zweite Filterretentat mit entionisiertem Wasser ergänzt wird.

15. Verfahren nach einem der Ansprüche 10 bis 14, wobei die Schritte c) - d) mindestens einmal wiederholt werden, um Ertrag und Reinheit zu verbessern.

16. Verfahren nach einem der Ansprüche 10 bis 15, wobei die Schritte c) - d) in einem geschlossenen Kreislaufsystem durchgeführt werden, das entweder konstant läuft oder während der Durchführung der spezifizierten Schritte konstant läuft.

17. Verfahren nach einem der Ansprüche 10 bis 16, wobei das Filtern den Vorgang der Dialyse einbezieht.

18. Verfahren nach einem der Ansprüche 10 bis 17, wobei in Schritt f) und/oder g) das zweite Filterretentat oder das erste Filtrat filtriert wird/werden, bis ein pH-Wert von weniger als 8 erreicht ist.

19. Verfahren nach einem der Ansprüche 10 bis 18, wobei das Kollagen aus Säugetier- oder Cnidaria-Gewebe extrahiert wird.

## Revendications

1. Collagènes isolés à chaîne alpha unique (SACC) en solution aqueuse, lesdits SACC étant ou ayant :
a) un polypeptide à chaîne unique existant sous la forme d'une hélice alpha et présentant un poids moléculaire de 80 à 120 kDa ;
b) une sous-structure répétitive de Glycine-X-Y (où X peut être n'importe quel acide aminé et Y est la proline ou l'hydroxyproline) ;
c) des extrémités N-terminales et/ou C-terminales non hélicoïdales ; et en outre
d) ladite solution aqueuse i) ne comprenant pas de collagène bêta ou gamma, ii) contenant une quantité de SACC par teneur totale en protéines de collagène d'au moins 75 % et iii) comprenant des SACC à une concentration d'au moins 85 % en poids.

2. SACC isolés selon la revendication 1, dans lesquels ladite solution aqueuse contient une quantité de SACC par teneur totale en protéines de collagène sélectionnée dans le groupe comprenant : au moins 85%, 86%, 87%, 88%, 89%, 90%, 91%, 92%, 93%, 94%, 95%, 96%, 97%, 98%, 99% ou 100%.

3. SACC isolés selon l'une quelconque des revendications 1 à 2, lesdits SACC étant d'origine mammalienne ou cnidarienne.

4. SACC isolés selon la revendication 3, lesdits SACC étant d'origine bovine, porcine, équine ou méduse.

5. SACC isolés selon l'une quelconque des revendications 1 à 4, lesdits SACC étant recombinants ou d'origine recombinante.

6. SACC isolés selon l'une quelconque des revendications précédentes, chaque SACC comprenant environ 1 000 à 3 500 acides aminés.

7. SACC isolés selon la revendication 6, chaque SACC comprenant environ 1 000 à 1 500 acides aminés.

8. SACC isolés selon l'une quelconque des revendications 1 à 7 destinés à être utilisés dans l'électrofilage.

9. Solution d'électrofilage comprenant les SACC isolés selon l'une quelconque des revendications 1 à 7 et une solution appropriée pour l'électrofilage.

10. Procédé de production ou de purification de collagène à chaîne alpha unique (SACC) comprenant :
a) l'obtention d'une extraction de collagène en solution alcaline ;
b) le filtrage de l'extraction à l'aide d'au moins une première membrane filtrante ayant un seuil de coupure du poids moléculaire nominal (NMWCO) dans la plage de 1 à 100 000 pour produire un premier rétentat de filtre ;
c) l'ajustement du pH du premier rétentat de filtre pour obtenir un pH acide inférieur à 3, solubilisant ainsi au moins partiellement le collagène lié par celui-ci ;
d) le filtrage du premier rétentat de filtre au moins partiellement solubilisé jusqu'à ce qu'un pH supérieur à 6 soit atteint à l'aide de ladite au moins une première membrane de filtre présentant un seuil de coupure de poids moléculaire nominal (NMWCO) dans la plage de 1 à 100 000 pour produire un second rétentat de filtre ;
e) l'ajustement du pH du second rétentat de filtre pour obtenir un pH inférieur à 3, solubilisant ainsi le collagène lié par celui-ci ;
f) le filtrage du second rétentat de filtre à l'aide d'au moins une seconde membrane filtrante dans laquelle la seconde membrane filtrante est une membrane microporeuse comprenant une taille de pore entre environ 0,05 et 2 µm et chaque nombre entier de 0,01 µm entre celles-ci pour produire un premier filtrat comprenant des SACC ; et
g) éventuellement, le filtrage du premier filtrat à l'aide de ladite au moins une première membrane filtrante pour produire un second second rétentat comprenant du collagène à chaîne alpha unique.

11. Procédé selon la revendication 10, dans lequel la première membrane filtrante présente un NMWCO choisi entre 10 000 et 50 000.

12. Procédé selon l'une quelconque des revendications 10 à 11, dans lequel à l'étape a), l'échantillon original contenant du collagène est traité avec une solution alcaline ou basique pour permettre la destruction de la matière cellulaire et d'autres matières non collagènes.

13. Procédé selon l'une quelconque des revendications 10 à 12, dans lequel à l'étape b), l'extraction est filtrée jusqu'à ce qu'un pH inférieur à 8 soit atteint et un premier rétentat de filtre soit obtenu.

14. Procédé selon l'une quelconque des revendications 10 à 13, dans lequel à l'étape b), le premier rétentat de filtre est complété, avant, pendant ou après le filtrage, avec de l'eau désionisée et/ou à l'étape d), le second rétentat de filtre est complété avec de l'eau désionisée.

15. Procédé selon l'une quelconque des revendications 10 à 14, dans lequel les étapes c) à d) sont répétées au moins une fois pour améliorer le rendement et la pureté.

16. Procédé selon l'une quelconque des revendications 10 à 15, dans lequel les étapes c) à d) sont réalisées dans un système circulatoire fermé qui fonctionne de manière constante ou qui fonctionne de manière constante pendant la réalisation desdites étapes spécifiées.

17. Procédé selon l'une quelconque des revendications 10 à 16, dans lequel ledit filtrage implique le processus de dialyse.

18. Procédé selon l'une quelconque des revendications 10 à 17, dans lequel à l'étape f) et/ou g), le second rétentat de filtre ou le premier filtrat est/sont filtré(s) jusqu'à ce qu'un pH inférieur à 8 soit atteint.

19. Procédé selon l'une quelconque des revendications 10 à 18, dans lequel ledit collagène est extrait d'un tissu mammalien ou cnidarien.
